# EUROPEAN PATENT SPECIFICATION

(11) **EP 3 972 675 B1**
(45) Date of publication and mention of the grant of the patent: **15.10.2025**
(21) Application number: 20810648.4
(22) Date of filing: 22.05.2020
(51) Int. Cl.: A61M 13/00, A61B 1/015

(54) **SYSTEM FOR SMOKE REMOVAL IN A GAS RECIRCULATION SYSTEM**
SYSTEM ZUR RAUCHENTFERNUNG IN EINEM GASRÜCKFÜHRUNGSSYSTEM
SYSTÈME D'ÉLIMINATION DE FUMÉE DANS UN SYSTÈME DE RECIRCULATION DES GAZ

(30) Priority: 23.05.2019 US 201962851950 P
(43) Date of publication of application: 30.03.2022
(73) Proprietor: Northgate Technologies Inc., Elgin, IL 60123 (US)
(72) Inventor: HABER, Brad, A., Hoffman Estates, IL 60192 (US); GUNDLAPALLI, Ramarao, Barrington, IL 60012 (US); BUMPUS, Jacob, M., Woodstock, IL 60098 (US); PUSH, Jason, T., Elgin, IL 60177 (US)
(74) Representative: Grünecker Patent- und Rechtsanwälte PartG mbB
(86) International application number: PCT/US2020/034288
(87) International publication number: WO 2020/237175

(56) References cited:
- WO-A1-2017/057030
- WO-A1-2017/057030
- US-A- 3 964 036
- US-A- 5 108 389
- US-A1- 2005 137 529
- US-A1- 2006 261 967
- US-A1- 2007 249 990
- US-A1- 2010 185 139
- US-A1- 2015 112 246
- US-A1- 2015 112 246
- US-A1- 2017 000 959
- US-B1- 6 203 590

## Description

### CROSS-REFERENCE TO RELATED APPLICATIONS

This application claims the benefit of US Provisional Patent Application Ser. No. 62/851,950, filed May 23, 2019.

### BACKGROUND

### 1. Technical Field text

The present disclosure relates to gas recirculation systems used in minimally invasive surgical procedures.

### 2. Background Information

Minimally invasive surgical procedures, including endoscopic surgical procedures, such as laparoscopic, arthroscopic, hyteroscopic, thoracoscopic surgical procedures, are becoming more common place in the surgical environment due to shorter recovery times, shorter operating durations, and reduced costs. Minimally invasive surgical procedures are typically performed with instruments inserted through small, artificially created openings or portals in the patient.

In a laparoscopic surgical procedure, a gas is injected into the peritoneal cavity through an artificial opening in the abdomen created by a verres needle. Typically, the type of gas that is injected is a CO₂ gas, although a mixture of two or more gases or a different gas may also be suitable depending on the surgical procedure. In a laparoscopic procedure, the CO₂ gas is used to distend the pneumoperitoneum, thereby creating an air space for the surgeon to visualize the organs and to maneuver surgical instruments and an endoscope. The CO₂ gas is injected into the peritoneal cavity under pressure by an insufflation device. Examples of insufflation devices suitable for this application are described in U.S. Patent No. 6,299,592 and U.S. Patent Serial No. 62/037893 or WO2017004490.

After the pneumoperitoneum is first distended, an endoscope with a camera (which is connected to a monitor) is inserted into the abdominal cavity to visualize the interior of the cavity and, more particularly, the operative space. The endoscope typically remains inserted for the duration of the surgical procedure. Other openings may also be created to provide access to other surgical instruments into the abdominal cavity.

The instrumentation used to cut, cauterize, ablate or vaporize tissues inside the abdomen during a minimally invasive surgical procedure, such as a laparoscopic procedure, results in surgical smoke which may pose a health risk to the patient and may also pose a health risk to the surgeon and other individuals in the operating room if some or all of the surgical smoke escapes to the operating room. As used herein, the term "surgical smoke" includes, without limitation, gases or aerosols that may contain toxins, particulate matter, irritants, viable cells and viruses, water vapor, and other contaminants. Surgical smoke also impairs the surgeon's visualization via the camera in the endoscope. This impairment to visualization can also be further accentuated by fogging or condensation on the camera lens due to the CO₂ gas entering the abdominal cavity at below body temperature. Impairing visualization can interfere with the surgical procedure and result in risk to the patient's health. Furthermore, impairing visualization may also lead to delays in the operation, in particular in operations involving robotic assisted surgical procedures performed remotely.

### BRIEF SUMMARY

In one aspect, a gas recirculation system for use in an endoscopic surgical procedure comprises a first tube in fluid communication with a gas input connection, where the first tube is connectable to surgical equipment that is insertable into a peritoneal cavity. A second tube is in fluid communication with a gas output connection, where the second tube is also connectable to surgical equipment that is insertable into a peritoneal cavity. The system also includes a pump having a motor to draw gas into the gas input connection from the peritoneal cavity through the first tube and to discharge gas out of the gas output connection and into a peritoneal cavity through the second tube. A smoke detection sensor is positioned at a location along a gas flow path defined by the first tube, the pump and the second tube and is configured to measure an amount of smoke present in the gas. The system additionally includes a controller configured to receive an output signal from the smoke detection sensor representative of an amount of smoke detected. The controller is further configured to adjust a speed of the motor of the pump in response to the amount of smoke detected. The motor speed may be increased in order to more quickly clear smoke from the peritoneum and filter the gas more quickly through one or more filter mechanisms in the gas recirculation system.

In other aspects, the gas recirculation system may also include a gas evacuation path that bypasses the recirculation system to more quickly remove smoke when the amount of smoke rises above a threshold manageable by the filters in the recirculation system. The smoke detection sensor may be located in or along the first tube, the second tube or the pump. In applications where the pump includes a removable pump cartridge, the sensor may be place on or inside he removable pump cartridge. Different sensor technologies such as electrochemical, optical and photoelectric sensors may be implemented in the smoke detection sensor. The smoke detection sensor may communicate over wired connections, or wirelessly, with the controller or pump of the gas recirculation system.

Other systems, methods, features and advantages will be, or will become, apparent to one with skill in the art upon examination of the following figures and detailed description. It is intended that all such additional systems, methods, features and advantages be included within this description, be within the scope of the invention, and be protected by the following claims.

### BRIEF DESCRIPTION OF THE DRAWINGS

FIG. 1 is an illustrative example of an embodiment of a gas recirculation system.
FIG. 2 is a schematic example of an embodiment of a gas recirculation system.
FIG. 3 is an example cross-section of an embodiment of a gas recirculation pump cartridge.
FIG. 4 is another example cross-section of an embodiment of a gas recirculation pump cartridge.
FIGS. 5A - 5D are an example of an embodiment of a gas recirculation pump cartridge.
FIGS. 6A - 6D are another example of an embodiment of a gas recirculation pump cartridge.
FIGS. 7A - 7H are an example of an embodiment of a portion of a gas recirculation pump cartridge.
FIGS. 8A - 8E are an example of an embodiment of a gas recirculation pump.
FIG. 9 is a block diagram of a gas recirculation system.
FIGS. 10A and 10B are an example of an embodiment of a coupling method between a gas recirculation pump cartridge and a motor.
FIGS. 11A - 11F are an example of an embodiment of a connecting element used in a gas recirculation system.
FIG. 12 is a cross-section of an embodiment of a connecting element used in a gas recirculation system.
FIG. 13 is a cross-section of another embodiment of a connecting element used in a gas recirculation system.
FIG. 14 is an example of an embodiment of a bypass valve used in a gas recirculation system.
FIGS. 15A and 15B are an example of an embodiment of a moisture trap used in a gas recirculation system.
FIGS. 16A and 16B are an example of another embodiment of a moisture trap used in a gas recirculation system.
FIGS. 17A - 17C are an example of an embodiment of an enclosure for a gas recirculation system.
FIGS. 18A and 18B are an example of another embodiment of an enclosure for a gas recirculation system.
FIGS. 19A and 19B are an example of another embodiment of an enclosure for a gas recirculation system.
FIGS. 20A - 20F are an example of another embodiment of an enclosure for a gas recirculation system.
FIGS. 21A - 21H are another example of an embodiment of a gas recirculation pump cartridge.
FIGS. 22A and 22B are an example of another embodiment of a coupling method between a gas recirculation pump cartridge and a motor.
FIGS. 23A - 23F are an example of another embodiment of a coupling method between a gas recirculation pump cartridge and a motor.
FIGS. 24A and 24B are an example of an embodiment of a three-way valve used in a gas recirculation system.
FIGS. 25A and 25B are an example of another embodiment of a three-way valve used in a gas recirculation system.
FIG. 26 is a schematic example of an embodiment of a gas recirculation system with smoke detection and venting capabilities.
FIG. 27 illustrates an smoke detection sensor for use in the system of FIG. 26.
FIG. 28 illustrates an alternative embodiment of the smoke detection sensor of FIG. 27.
FIG. 29 is a schematic of an alternative embodiment of the gas recirculation system of FIG. 26.
FIG. 30 is an embodiment of a gas pump cartridge with photoelectric smoke sensor usable in the gas recirculation system of FIG. 29.
FIG. 31 illustrates an array of photoelectric detectors usable in the smoke sensor of the gas pump cartridge of FIG. 30.
FIG. 32 illustrates an electrochemical smoke detector for use in a gas recirculation system.
FIG. 33 is an embodiment of a gas pump cartridge with electrochemical smoke sensor usable in the gas recirculation system of FIG. 29.
FIG. 34 illustrates a motor enclosure that may be connected with the removable pump cartridge of FIG. 33.
FIG. 35 is an alternative embodiment of the motor assembly of FIG 22A, usable with the motor enclosure of FIG. 34 and modified with electrical connectors to receive the gas pump cartridge of FIG. 33
FIG. 36 is an alternative embodiment of the gas pump cartridge of FIG. 33.
FIG. 37 illustrates a smoke sensor positioned in an input male Luer valve of the gas recirculation pump tubing set.
FIG. 38 illustrates a smoke sensor positioned in an output male Luer valve of the gas recirculation pump tubing set.
FIG. 39 illustrates a method of adjusting the speed of a pump based on smoke detection criteria to change a rate of smoke removal in the system of FIGS. 26 or 29.
FIG. 40 illustrates a valve for switching between a gas recirculation path and a suction exhaust path in the system of FIG. 26.
FIG. 41 illustrates a method of adjusting a speed of gas recirculation, or alternatively venting gas from a gas recirculation system, in the system of FIG. 26 based on an amount of smoke detected in the gas.

### DETAILED DESCRIPTION

The present disclosure is directed to a system for recirculating gas injected into a peritoneal cavity during a surgical procedure. The system includes a positive displacement pump to remove and inject gas into the peritoneal cavity in order to remove smoke generated within the peritoneal cavity during the surgical procedure.

In order to address the challenges of filtering, or otherwise removing, smoke from the peritoneum during a surgical procedure, a system and method for detecting an amount of smoke and automatically adjusting the process of filtering and recirculating gases based on the detected amount of smoke is described herein. The systems and methods described may provide a rapid response to handling removal of smoke generated in the peritoneum that does not require manual adjustment or intervention and is independent of the type of surgery.
In different implementations, a gas recirculation system according to the invention will sense a density and quantity of smoke within the pneumoperitoneum and correlate that information to the visibility within the cavity. Any number of types of smoke detection sensors, such as a sensor using light sensing technology, may be used to determine the density of smoke. Also, the sensor may either be placed within the pneumoperitoneum or in-line with a tubing set used for CO₂ (or other gas type) evacuation or circulation. The system may then use the determined density of smoke to increase or decrease the CO₂ movement within the peritoneal cavity to quickly remove the smoke.

The present disclosure provides a safe, cost effective gas recirculation system with component parts that can be reused without sterilization. The cost effective system utilizes controllers, rather than sensors, to monitor the pump operation and detect faults. The system is able to achieve high removal and injection flow rates, for example 4 to 10 liters per minute, which ensures that any surgical smoke is quickly and effectively removed from the surgeon's field of vision while at the same time minimizing any change in pressure in the peritoneal cavity.

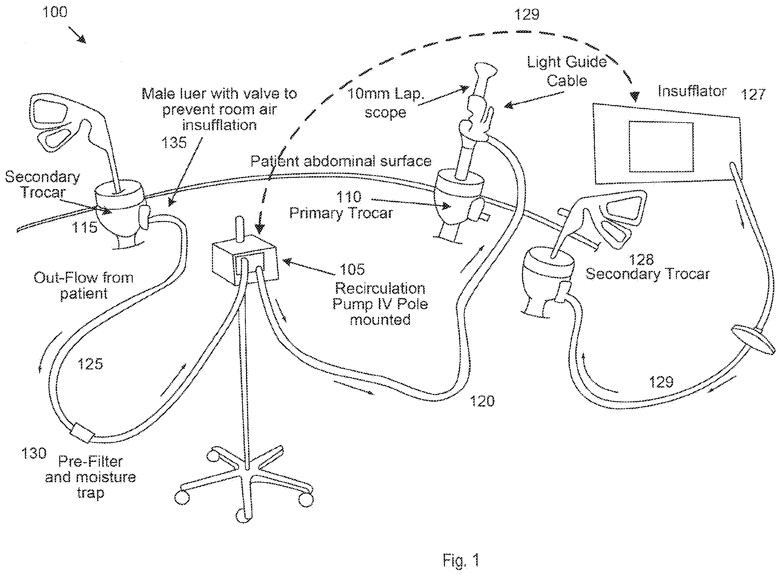

Referring to FIG. 1, an embodiment of a gas recirculation system 100 is shown. Gas recirculation system 100 may include a recirculation pump 105, a primary input trocar 110, a secondary output trocar 115, input tubing 120, and output tubing 125. Output tubing 125 may include a filter and/or moisture trap 130. Input tubing 120 and output tubing 125 may be similar to insufflation tubing sets manufactured by Northgate Technologies.

Gas recirculation system 100 may be used in conjunction with an insufflation system, such as described in U.S. Patent No. 6,299,592 and U.S. Patent Serial No. 62/037893. The insufflation system may include an insufflator 127, an insufflation trocar 128, insufflation tubing 129 connecting the insufflator 127 to the insufflation trocar 128, and an electronic communication line 129 between gas recirculation system 100 and insufflator 127. Gas recirculation system 100 may include a controller to communicate with insufflator 127 through communication line 129. Information or commands such as start, stop, flow increase, flow decrease, or other functions of the gas recirculation controller could reside in insufflator 127 and communicated to the gas recirculation controller. Additionally or alternatively, the gas recirculation controller could be integrated into and shared with insufflator 127. The gas recirculation system 100 and insufflator 127 may share power supplies, processors, graphic user interfaces, heat functions, humidity functions, to name a few examples.

Recirculation pump 105 removes gas from the patient through secondary output trocar 115, output tubing 125, and filter/moisture trap 130. A valve 135 may connect secondary output trocar 115 to output tubing 125. When output tubing 125 is connected to secondary output trocar 115 through valve 135, the valve stem of valve 135 may be deflected to an open position. When valve 135 is disconnected from secondary output trocar 115, the valve stem of valve 135 may return to its natural closed position. Valve 135 may allow gas to flow through the valve when output tubing 125 is connected to secondary output trocar 115. Valve 135 may prevent gas from entering output tubing 125 when output tubing 125 is disconnected from secondary output trocar 115. Valve 135 may automatically close when output tubing 125 is disconnected from secondary output trocar 115. Valve 135 may be a luer valve, such as a Texium^{®} or Halkey/Roberts^{®} brand of closed male luers.

Recirculation pump 105 also injects gas into the patient through primary input trocar 110 and input tubing 120. A valve, similar to valve 135, may connect primary input trocar 110 and input tubing 120 and may close when input tubing 120 is disconnected from primary input trocar 110.

Recirculation pump 105 recirculates gas from the peritoneal cavity, through filter/moisture trap 130, and back into the peritoneal cavity. The flow rate of gas removed from the patient through output tubing 125 is the same as or substantially similar to the flow rate of gas injected back into the patient through input tubing 120. Filter/moisture trap 130 may remove liquid from the gas and may remove particulate from the gas, such as surgical smoke particles. Filter/moisture trap 130 may include a media that readily absorbs liquid, preferably up to 15 to 20 ml of liquid, and readily releases moisture into the gas flowing over or through the media. A media that is suitable for use includes the Crystar^{®} brand of material. The size of the media is preferably 1 - 2.5 inches long and 0.5 - 2.0 inches in diameter and most preferably 1.5 - 2.0 inches long and 1 - 1.5 inches in diameter. In one embodiment, the media may have a serrated outer surface and a center opening. When placed in a filter housing, the serrated outer surface defines a plurality of channel openings in which the gas can flow and the center opening may be filled with a rod comprising charcoal. The charcoal may entrap particulate matter in the gas as it passes through the center opening and at the same time may be effective at removing undesirable odor from the gas. Additionally or alternatively, odor removal can be accomplished using other materials, such as enzymatic materials, vinegar and water cartridges, or odor can be masked using fragrances. Filter/moisture trap 130 may allow the gas that is recirculated to retain moisture in a range of 50 - 70% relative humidity. Preferably, the gas recirculation system 100 will allow for the recirculation of gas to and from the patient and will passively maintain a humidity level of the gas that will be a minimum of 70% relative humidity with the gas at normal operating room temperatures between 60 - 75 degrees Fahrenheit. Utilizing gas recirculation system 100 may reduce or eliminate the need for insufflator 127 to inject additional CO₂ gas in the peritoneal cavity and may also maintain a reasonable moisture level in the peritoneal cavity, as opposed to added CO₂ which, unless it first passes through a gas warmer humidifier (an additional cost) will be very dry, typically at 0% relative humidity. The recirculation of gas will not only reduce the input of 0% relative humidity gas, but also may prevent the breathing effect caused by insufflator 127 attempting to maintain pressure in the peritoneal cavity, and prevent the discharge of large amounts of CO₂ gas into the operating room. For example, passive smoke removal systems that allow six liter per minute leak rates may discharge up to 270 liters of CO₂ gas into the operating room during a normal 45 minute gall bladder procedure. Accordingly, gas recirculation system 100 is a cost effective method to maintain adequate humidity of gas in the peritoneal cavity.

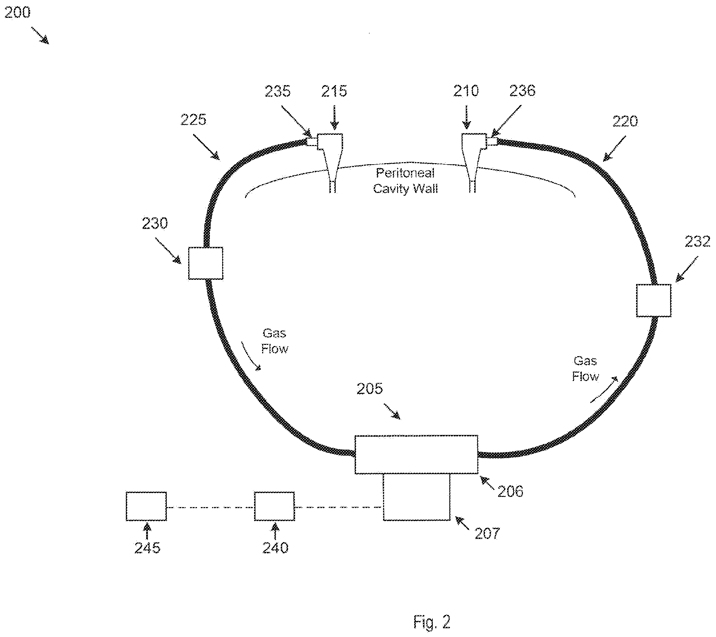

Referring to FIG. 2, an embodiment of a gas recirculation system 200 is shown. Gas recirculation system 200 may include some of the same components and operational characteristics as gas recirculation system 100. Gas recirculation system 200 may include a recirculation pump 205, an input trocar 210, an output trocar 215, input tubing 220, and output tubing 225. Output tubing 225 may include a filter and/or fluid trap 230. Input tubing 220 may include a filter 232. A valve 235 may connect output trocar 215 to output tubing 225. A valve 236 may connect input trocar 210 to input tubing 220. Valves 235, 236 may operate with the same characteristics, such as automatically closing when disconnected, and in the same manner as valve 135.

Recirculation pump 205 may be a diaphragm pump, or any other suitable positive displacement pump, including a cartridge 206 and a motor 207. Cartridge 206 may be disconnectable from motor 207. Motor 207 may be any type of motor. Motor 207 may preferably be, but not limited to, a direct current ("DC") motor. Cartridge 206 may be sealed to prevent gas from escaping cartridge 206 except through the connection to input tubing 220 and outlet tubing 225. Cartridge 206 may be composed of multiple components that are attached to one another, such as by ultrasonic welding, using adhesives, laser welding, mechanical snapping connection with or without a gasket, or any other known method of combining and sealing mating surfaces together. Cartridge 206 may be sealed so that it is only in fluid communication with the opening to inlet tubing 220 and outlet tubing 225. Accordingly, gas within cartridge 206 may not come in contact with motor 207 or other parts of recirculation pump 205. The gas recirculation system may be an inexpensive method to remove surgical smoke from a patient's peritoneal cavity because motor 207 is not contaminated from contact with gas from the peritoneal cavity, and therefore, can be reused without requiring sterilization. The portions of recirculation pump 205 that may have been contaminated from contact with gas from the peritoneal cavity, such as cartridge 206, may be disposable.

When in operation, the gas recirculation system 200 may remove gas, including surgical smoke, from a peritoneal cavity preferably at a flow rate of 4 - 10 liters per minute and most preferably at a flow rate of 6 - 8 liters per minute and, after filtration, inject it back into the peritoneal cavity preferably at a flow rate of 4 - 10 liters per minute and most preferably at a flow rate of 6 - 8 liters per minute. The gas from the peritoneal cavity first travels through output trocar 215, through valve 235, and into output tubing 225. The gas may travel through fluid trap 230 which may remove condensate / liquid that forms due to the change in temperature of the gas (i.e. from body temperature to room temperature) and odor if a charcoal rod, (as described above) or a separate or integrated activated charcoal filter is used. The gas then travels through cartridge 206 of recirculation pump 205. The gas may travel through a filter that is located before or after recirculation pump 205, such as filters 230 or 232. The filter may remove particulate matter and other contaminants from the gas. The filter is preferably is made of a material that provides a pressure drop of no more than 12.3 mmHG at a 20 liter per minute flow rate. The gas may be injected back into the peritoneal cavity through input tubing 220, valve 236, and input trocar 210.

Recirculation system 200 may include controller 240 to control the operation of motor 207. Controller 240 may be combined with or used in conjunction with an insufflator connected to recirculation system 200. Controller 240 may be the Tiva^{®} (Texas Instruments) brand of controllers. Controller 240 may be used to detect operating and fault conditions of motor 207 and/or safety issues in gas recirculation system 200. Controller 240 may detect the amount of power drawn by motor 207, such as by measuring the voltage to motor 207. Controller 240 may detect or determine that a fault or safety issue has occurred in gas recirculation system 200 based on the amount of power drawn by motor 207. For example, controller 240 may determine a fault condition or safety issue occurs if motor 207 draws more power than expected, as measured by an increase in voltage or current greater than a predetermined amount. Controller 240 may trigger a shutdown of motor 207 if a fault condition or safety issue occurs. Using controller 240 to detect fault conditions or safety issues in gas recirculation system 200 may be more cost effective than using sensors.

Valves 235 and 236 may be configured to close if they are disconnected from output trocar 215 and input trocar 210, respectively. Closing valve 235 when it is disconnected from output trocar 215 may restrict entrainment of ambient air into the suction side of gas recirculation system 200. Any ambient air entrained in gas recirculation system 200 would be injected into the peritoneal cavity by recirculation pump 205. Closing valve 236 when it is disconnected from input trocar 210 may prevent discharging gas from the peritoneal cavity into the ambient environment.

Closing valves 235 or 236 may create a pressure differential in the gas circuit of gas recirculation system 200. A pressure differential may increase the load on motor 207, as measured by an increase in voltage or current drawn by motor 207. If the increase in voltage or current is above a predetermined threshold value, controller 240 may detect a fault condition or safety issue in gas recirculation system 200. Controller 240 may trigger a shutdown of motor 207 upon detection of a fault condition or safety issue in gas recirculation system 200. For example, valve 235 will close if valve 235 and output tubing 225 are disconnected from output trocar 215. Closing valve 235 will cause recirculation pump 205 to pull suction on a closed tube, which will force recirculation pump 205 to work harder and motor 207 to draw more power in order to maintain its proper speed. The increase in power drawn by motor 207 may result in a fault condition if the voltage or current increase is above a predetermined value. Upon detection of the fault condition caused by disconnecting valve 235 from output trocar 215, controller 240 may trigger recirculation pump 205 to shut down. Similarly, valve 236 will close if valve 236 and input tubing 220 are disconnected from input trocar 210. Closing valve 236 will cause recirculation pump 205 to pump against a closed tube or "dead head," which will force recirculation pump 205 to work harder and motor 207 to draw more power in order to maintain its proper speed. The increase in power drawn by motor 207 may result in a fault condition if the voltage or current increase is above a predetermined value. Upon detection of the fault condition caused by disconnecting valve 236 from input trocar 210, controller 240 may trigger recirculation pump 205 to shut down. Accordingly, gas recirculation system 200 may monitor the status of output tubing 225 and input tubing 220 by using controller 240 to monitor motor 207.

In a similar manner, gas recirculation system 200 may monitor the connection status of input trocar 210 and output trocar 215 with the peritoneal cavity. Removing input trocar 210 or output trocar 215 from the peritoneal cavity will affect the operation of recirculation pump 205 and motor 207 by changing the pressure of the suction source or discharge source of recirculation pump. Controller 240 may detect the change in operation of the motor 207 and determine that input trocar 210 or output trocar 215 has been removed from the peritoneal cavity. For example, removing input trocar 210 from the peritoneal cavity would decrease the power required for motor 207 to maintain the same speed because recirculation pump 205 would no longer be pumping to overcome the intraperitoneal pressure. Controller 240 may detect the decreased power drawn by motor 207 and determine that input trocar 210 has been disconnected from the peritoneal cavity. Controller 240 may then trigger recirculation pump 205 to shutdown to prevent gas from the peritoneal cavity entering the ambient environment.

Gas recirculation system 200 may include a user interface 245, such as a computer, to allow an operator to determine or confirm the status of gas recirculation system 200. For example, if controller 240 shuts down recirculation pump 205 because valve 235 is disconnected from output trocar 215, user interface 245 may display that recirculation pump 205 is shut down and that the likely cause is output tubing 225 being disconnected from output trocar 215. An operator may confirm that output tubing 225 is disconnected from output trocar 215 and reconnect it in order to restart recirculation pump 205. Similarly, an operator may determine if other fault conditions have occurred, such as blockage, excessive restriction in the gas path, or a leakage in the gas path, such as disconnected or damaged tubing.

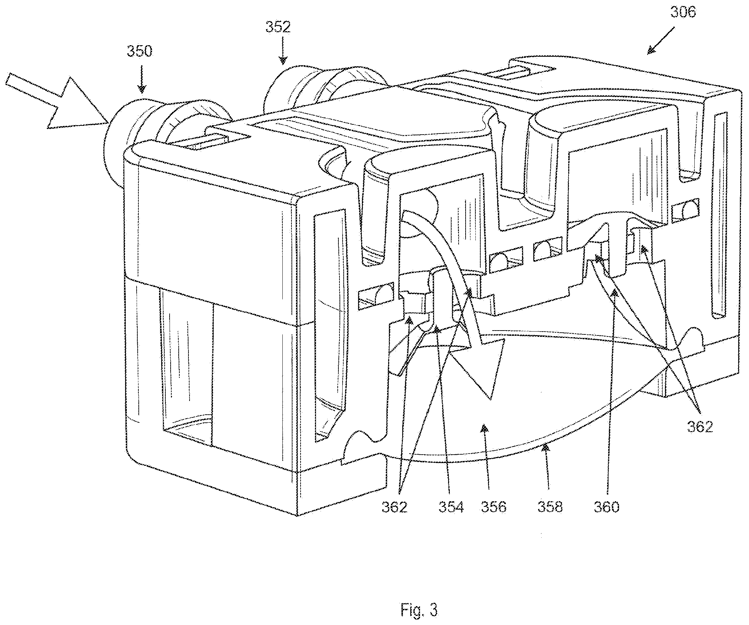

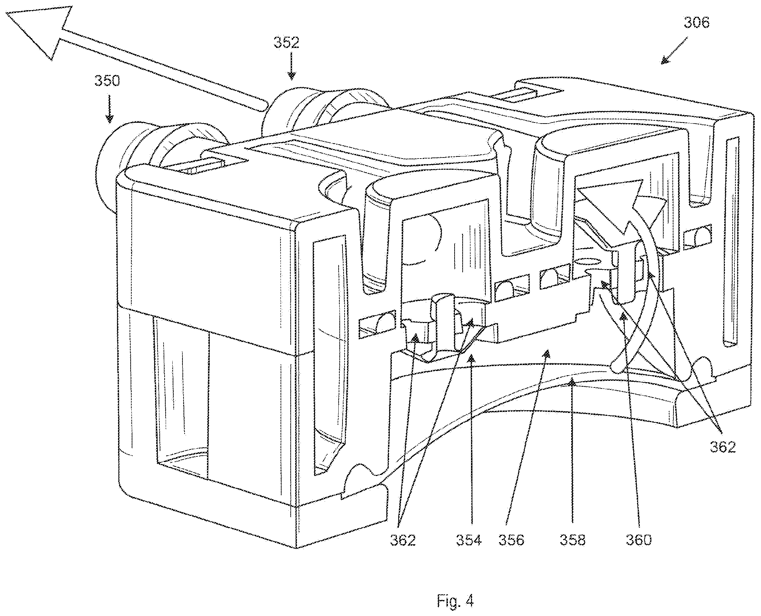

Referring to FIG. 3 and FIG. 4, an embodiment of a cartridge 306 used in a recirculation pump is shown. Cartridge 306 may be used in a recirculation pump such as recirculation pump 205 described in relation to FIG. 2. FIG. 3 and FIG. 4 show a partial cross-sectional view of cartridge 306. Arrows showing the gas flow path are included in order to better describe the operation of cartridge 306. Cartridge 306 includes connection 350 to output tubing, such as output tubing 225 in FIG. 2, that may be connected to a peritoneal cavity. Cartridge 306 includes connection 352 to input tubing, such as input tubing 220 in FIG. 2, that may be connected to a peritoneal cavity.

Gas from the peritoneal cavity enters cartridge 306 through connection 350, as shown by the arrow in FIG. 3. Cartridge 306 may include valves 354 and 360. The gas travels into cartridge 306 through valve 354 into diaphragm chamber 356, as shown by the arrow in FIG. 3. The gas travels out of cartridge 306 from diaphragm chamber 356 through valve 360, as shown by the arrow in FIG. 4 (discussed below). Valves 354 and 360 may be umbrella valves. The diameter of the gas opening 362 through valves 354 and 360 may be between 0.05 inches and 0.15 inches and may be preferably a diameter of 0.085 inches. The gas openings 362 may include more than one concentric opening, such that the combined area of the gas openings 362 may be sized to permit different flow rates. For example, the openings may be sized for a flow rate within a range of 4 liters per minute up to 10 liters per minute and a first preferred range of 7 to 8 liters per minute. Although flow rates of 4 - 10 liters per minute are an acceptable flow range, higher or lower flow rates can be achieved by enlarging or reducing the size of the cartridge, increasing or decreasing the motor stroke length to change the volume created within the diaphragm cavity, or by increasing the speed of the motor. For example, in another embodiment, a second preferred flow rate range of 10-12 liters per minute may be implemented via adjusting one or more of gas opening size, cartridge size, motor stroke length or motor speed. The higher flow rate range of 10-12 liters per minute may be preferred when implementing smoke sensing and filtration/evacuation techniques described below.

Cartridge 306 may include a diaphragm 358 in diaphragm chamber 356. Movement of diaphragm 358 away from valves 354 and 360 opens valve 354 and draws gas through valve 354 into diaphragm chamber 356, as shown by the arrow in FIG. 3. Valve 354 may be pulled open when diaphragm 358 moves away from valves 354 and 360, which may draw gas from the peritoneal cavity, through output tubing and into diaphragm chamber 356, as shown by the arrow in FIG. 3. Valve 360 may be pulled closed when diaphragm 358 moves away from valves 354 and 360, which may prevent gas from exiting or entering diaphragm chamber 356 through valve 360.

Movement of diaphragm 358 toward valves 354 and 360 opens valve 360 and pushes gas from diaphragm chamber 356, through valve 360 and out of cartridge 306 through connection 352, as shown by the arrows in FIG. 4. Movement of diaphragm 358 toward valves 354 and 360 closes valve 354, which may prevent pushing gas out of diaphragm chamber 356 through connection 350. Reciprocal movement of diaphragm 358 toward and away from valves 354 and 360 draws gas from the peritoneal cavity, through any filter or liquid trap in the output tubing, and pushes gas back into the peritoneal cavity through the input tubing.

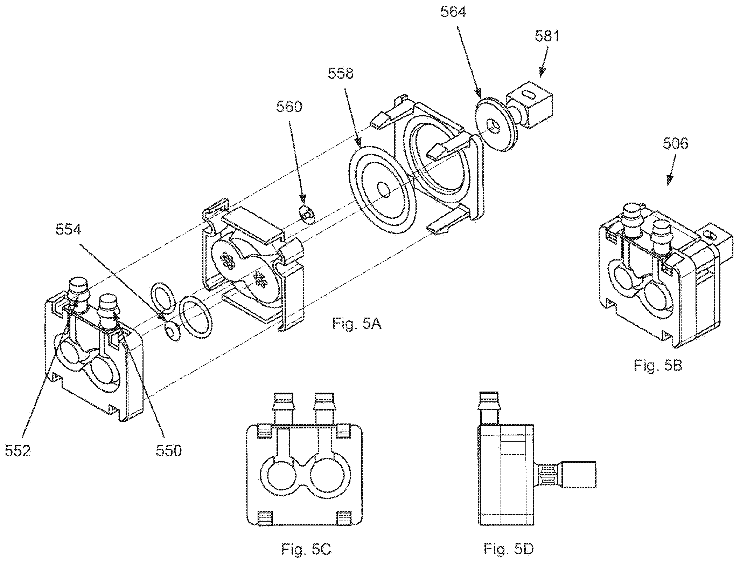

FIGS. 5A-5D, 6A-6D, and 7A-7H show other example embodiments of cartridges for use in a gas recirculation pump, such as recirculation pump 205 described in relation to FIG. 2. The components and operational characteristics of the cartridges shown in FIGS. 5A-5D, 6A-6D, and 7A-7H may be similar to cartridge 306, described above.

FIG. 5A shows an exploded view of cartridge 506. Cartridge 506 includes connections 550, 552, valves 554, 560, diaphragm 558, and plunger 564. Valves 554, 560 may be umbrella valves. Connection 550 may be the gas inlet into cartridge 506. Connection 552 may be the gas outlet from cartridge 506. Plunger 564 may move diaphragm 558 toward valves 554, 560 in order to recirculate gas through the peritoneal cavity, as described above in reference to FIGS. 3 and 4.

FIG. 5B shows a non-exploded perspective view of cartridge 506. FIG. 5C shows a front view of cartridge 506. FIG. 5D shows a side view of cartridge 506.

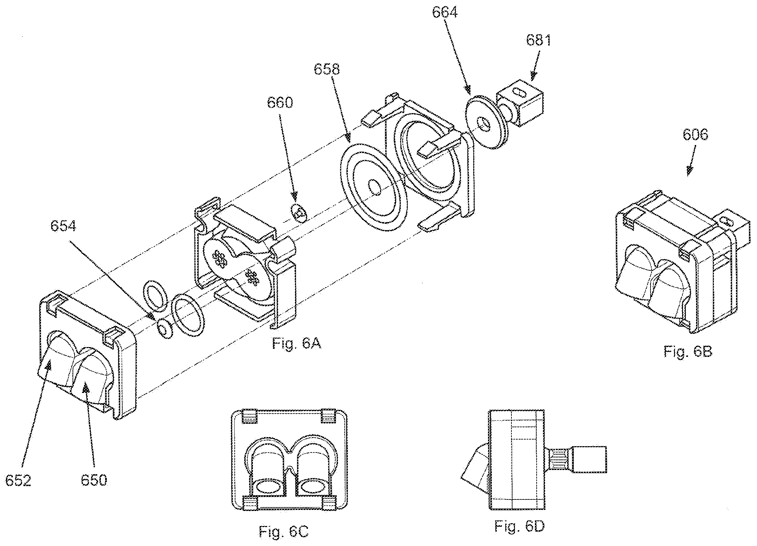

FIG. 6A shows an exploded view of cartridge 606. Cartridge 606 includes connections 650, 652, valves 654, 660, diaphragm 658, and plunger 664. Valves 654, 660 may be umbrella valves. Connection 650 may be the gas inlet into cartridge 606. Connection 652 may be the gas outlet from cartridge 606. Plunger 664 may move diaphragm 658 toward valves 654, 660 in order to recirculate gas through the peritoneal cavity, as described above in reference to FIGS. 3 and 4.

FIG. 6B shows a non-exploded perspective view of cartridge 606. FIG. 6C shows a front view of cartridge 606 with exemplary dimensions. FIG. 6D shows a side view of cartridge 606 with exemplary dimensions. The dimensions and orientations of the components of cartridge 606 may vary depending on operational requirements.

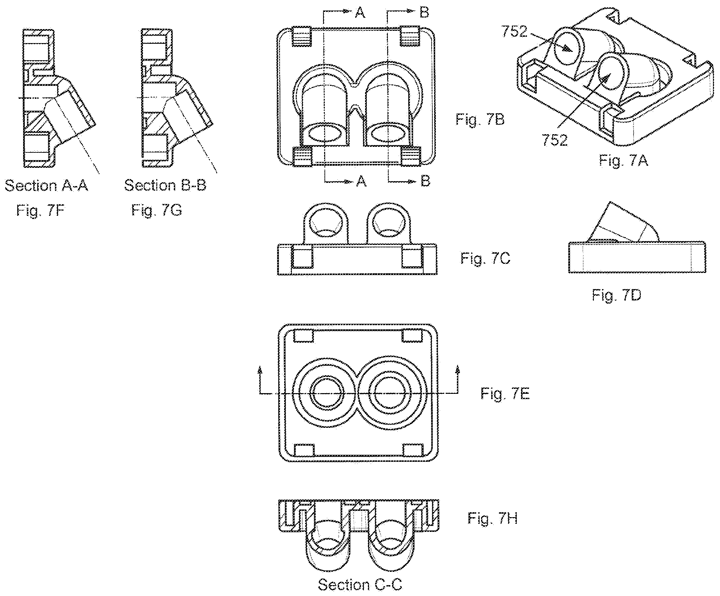

FIGS. 7A-7H show multiple views of the gas inlet/outlet section of cartridge 706. FIG. 7A shows a perspective view of the gas inlet/outlet section of cartridge 706. FIG. 7B is a front view of the gas inlet/outlet section of cartridge 706. FIG. 7C is a bottom view of the gas inlet/outlet section of cartridge 706 with exemplary dimensions. FIG. 7D is a side view of the gas inlet/outlet section of cartridge 706. FIG. 7E is a back view of the gas inlet/outlet section of cartridge 706 with exemplary dimensions. FIG. 7F is a side cross-sectional view of the gas inlet/outlet section of cartridge 706 with exemplary dimensions. FIG. 7G is another side cross-section view of the gas inlet/outlet section of cartridge 706 with exemplary dimensions. FIG. 7H is bottom cross-section view of the gas inlet/outlet section of cartridge 706. The dimensions and orientations of the components of cartridge 706 may vary depending on operational requirements.

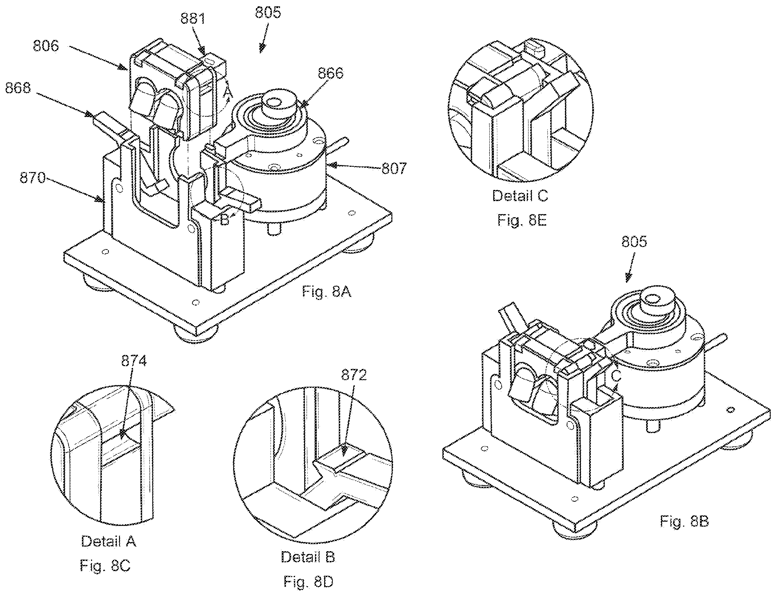

Referring to FIGS. 8A-8E, an embodiment of a recirculation pump 805 is shown. Recirculation pump 805 may include a cartridge 806, a motor 807, a crank assembly 866, locking arms 868, and a cartridge holder 870. The components and operational characteristics of recirculation pump 805 may be similar to recirculation pump 305, described above. Motor 807 may be connected to crank assembly 866 through a mechanical coupling. Motor 807 may provide rotational motion to crank assembly 866. Crank assembly 866 may convert the rotational motion to a reciprocal motion. The reciprocal motion of crank assembly 866 may move a diaphragm within cartridge 806, as described above with reference to FIG. 3. FIG. 8A shows cartridge 806 detached from recirculation pump 805. Cartridge 806 may be detached from recirculation pump 805 in order to sterilize or dispose of cartridge 806. Because cartridge 806 may be the only component of recirculation pump 805 that comes in contact with gas from a patient's peritoneal cavity, the remaining components of recirculation pump 805 may be reused with a different patient without risking patient safety. Cartridge 806 may be sterilized or disposed of after use with a patient and a new cartridge 806 may be inserted into recirculation pump 805 for the next patient.

FIG. 8B shows cartridge 806 inserted into cartridge holder 870 of recirculation pump 805. Cartridge 806 may be secured within recirculation pump 805 with locking arms 868. Locking arms 868 may include protrusions 872 designed to fit within recesses 874 located in cartridge 806. Protrusions 872 may be best seen in FIG. 8D. Recesses 874 may be best seen in FIG. 8C. Cartridge 806 may be secured within cartridge holder 870 when protrusions 872 are placed in recesses 874, as shown in FIG. 8F. Cartridge 806 may be released from cartridge holder 870 by depressing the ends of locking arms 868 and then lifting cartridge 806 from cartridge holder 870. The method of securing and releasing cartridge 806 from recirculation pump 805 may vary depending on operational requirements.

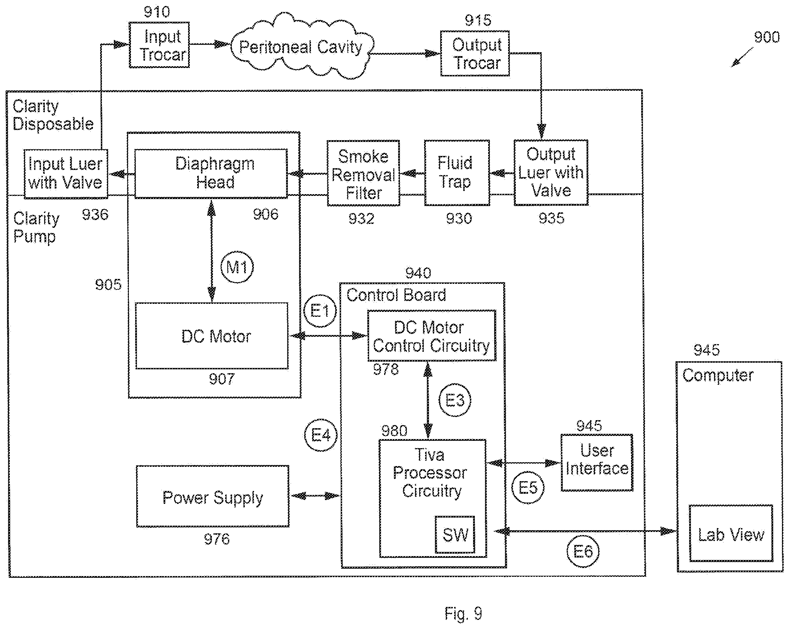

Referring to FIG. 9, an embodiment of a gas recirculation system 900 is shown. Gas recirculation system 900 may include similar components and operating characteristics as the gas recirculation systems described in FIGS. 1-8. Gas recirculation system 900 may include recirculation pump 905, pump cartridge 906, motor 907, input trocar 910, output trocar 915, valves 935 and 936, fluid trap 930, filter 932, controller 940, user interface 945, and power supply 976. Controller 940 may include DC motor control circuitry 978 and processor circuitry 980. User interface 945 may include a computer with software, such as LabView^{®}, to control some or all components of gas recirculation system 900.

Gas recirculation system 900 may monitor the load placed on motor 907 in order to detect faults or safety issues with gas recirculation system 900. The load on motor 907 may be monitored by measuring the current change across a resistor located in the power path of motor 907, such as by connecting the resistor to an A-D converter to measure the current. The current will change as the load on motor 907 changes. The current measurement may be measured in real time or may include a delay. A change in current above or below a predetermined value may indicate that gas recirculation system 900 has a fault or safety issue and may initiate a shutdown of recirculation pump 905. Software may be included, for example in controller 940, to sense a change in current and to initiate a shutdown of motor 907.

The predetermined value of current that defines when a fault or safety issue occurs may be based on an average current when gas recirculation system 900 is operating normally. A current measurement above the average value may indicate a fault or safety condition, such as a disconnected valve 935 or 936 or an occlusion in the tubing connecting recirculation pump 905 to a patient's peritoneal cavity. For example, if the average current measured while motor 907 was driving a diaphragm in cartridge 906 during normal operation was 0.3A, a measured current of 0.4A may indicate an occlusion in the tubing connecting recirculation pump 905 to the patient's peritoneal cavity and a measured current of 0.5A may indicate one of valves 935 or 936 were disconnected. Other methods or statistics could be used to define when a fault or safety condition occurs, such as by using a variance of measured currents or a comparison against a stored time template or frequency template. Additionally or alternatively, a processor in controller 940 may be capable of a Fast Fourier Transform to analyze the frequency content of the current measurement signal.

Interface M1 between pump cartridge 906 and motor 907 may be a mechanical interface. Interface M1 may be designed to operate adequately for continuous periods of time greater than the length of time gas recirculation system is used during a surgical procedure. For example, if the maximum length of time for a surgical procedure is four hours, interface M1 may be designed to operate continuously without error for eight hours.

The speed of motor 907 may be specified to allow the delivery of CO₂ gas at a rate of seven liters per minute. A motor suitable for motor 907 may include a Moog^{®} brand high speed motor. The key operating parameters for motor 907 may be the torque, speed, and fault conditions. The operating current of motor 907 may be specified in several ways, such as the normal operating current, the fault current, the inflate state current, and the deflate state current. These current values may be used to define when motor 907 should be shut down due to a fault or safety condition.

Interface E1 is between motor 907 and DC motor control circuitry 978. There may be eight lines in interface E1. The eight lines may include a line for each of the three drive phases of motor 907, a line each for three hall sensor pickups, a line to power the hall sensors, and a line for a ground. These eight lines may be common to multiple motor manufacturers.

Interface E3 is between DC motor control circuitry 978 and processor circuitry 980. There may be multiple lines in this interface depending on the method of speed control and feedback.

The speed of motor 907 may be controlled using two methods: voltage and digital control of the motor. The first method using voltage control would result in the processor circuitry 980 sending a voltage to the control circuitry 978 via a potentiometer or pulse width modulated signal. For reference, in this method the full speed of motor 907 may be reached by having the processor circuitry 980 provide the voltage of 3.25V to the motor control circuitry. The second method would involve in the processor circuitry 980 sending a digital signal to the motor control circuitry 978.

Gas recirculation system 900 may detect two fault states that are recoverable, such as the inflate fault state and the deflate fault state. Other fault states may occur that are not recoverable, such as a problem with motor 907. The inflate fault state may be when the gas circuit on the suction side of gas recirculation pump 905 is broken such that ambient air is drawn into gas recirculation system 900, for example if valve 935 is disconnected from output trocar 915. Such a state is named "inflate" because recirculation pump 905 may inflate the patient's peritoneal cavity with ambient air if recirculation pump 905 is not shutdown. An alternative to shutting down recirculation pump 905 if an inflate fault state occurs may be to reduce the gas flow through recirculation pump 905 to a small amount in order to minimize the amount of ambient air pumped into the peritoneal cavity. The deflate fault state may be when the gas circuit on the discharge side of gas recirculation pump 905 is broken such that gas from the peritoneal cavity is pumped into the ambient environment, for example if valve 936 is disconnected from input trocar 910. Such a state is named "deflate" because the peritoneal cavity may begin to deflate due to the loss of gas from gas recirculation system 900. A deflate fault state may cause the activation of an insufflator connected to the peritoneal cavity in order to maintain a desired inflation level or pressure in the peritoneal cavity.

Gas recirculation system 900 may be controlled through user interface 945. User interface 945 may be located in gas recirculation system 900 and/or in a computer connected to gas recirculation system 900. User interface may be multimode interface which may be controlled by software, such as LabView^{®}. The first mode may be Output and the second mode may be Control. In Output mode, the processor in controller 940 may output information regarding monitoring motor 907. Such information may include motor speed (RPM), current (mA), voltage (V), and motor state.

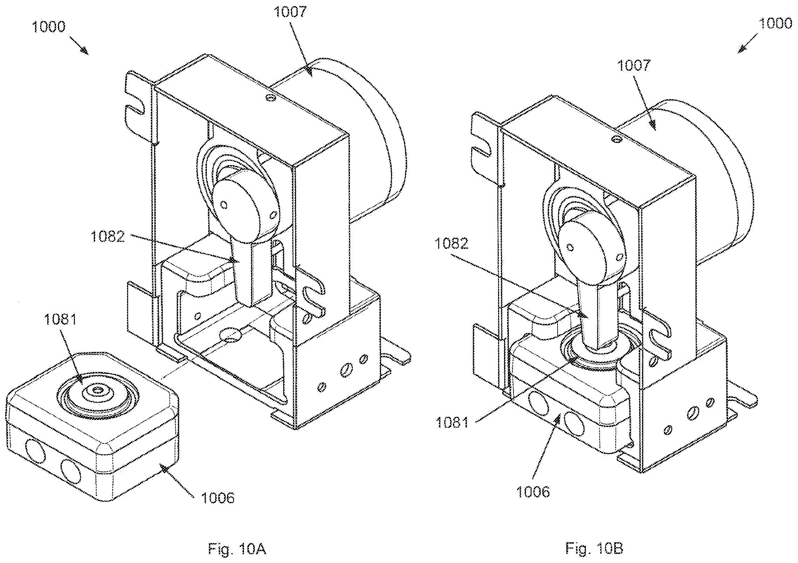

Referring to FIGS. 10A-10B, an embodiment of a gas recirculation system 1000 is shown. Gas recirculation system 1000 may include similar components and operating characteristics as the gas recirculation systems described in FIGS. 1-9. Gas recirculation system 1000 may include a magnetic coupling between the diaphragm actuator 1081 and the motor coupling arm 1082 such that when the pump cartridge 1006 is inserted into position, a magnet on the diaphragm actuator 1081 is drawn to a magnet on the motor coupling arm 1082. Once the magnets are drawn together, the diaphragm actuator 1081 will follow the motor coupling arm 1082 up and down, causing pumping action in the pump cartridge 1006 (as discussed above), as the motor coupling arm 1082 moves up and down. The magnetic coupling may be an electromagnet that is cycled on and off to create and release the coupling between the diaphragm actuator 1081 and the motor coupling arm 1082, such as for removal of the pump cartridge 1006. Alternatively, the magnetic coupling may be a non-electromagnet. FIG. 10A shows the pump cartridge 1006 with a magnet on the diaphragm actuator 1081 before it is inserted and coupled to the motor coupling arm 1082. FIG. 10B shows the pump cartridge 1006 after it is inserted and the diaphragm actuator 1081 is magnetically coupled to the motor coupling arm 1082.

Alternatively, rather than using a motor with a crank arm to move the diaphragm actuator 1081 up and down, an oscillating magnetic field could be used to move a magnet attached to or embedded in the diaphragm actuator 1081 in order to move the diaphragm actuator 1081 up and down and create a pumping action in the pump cartridge 1006. Additionally or alternatively, a spring located within the pump cartridge 1006 could provide upward motion of the diaphragm, while a motor with a crank arm could provide the downward motion. Such an arrangement may eliminate the need to couple the diaphragm with the motor crank arm.

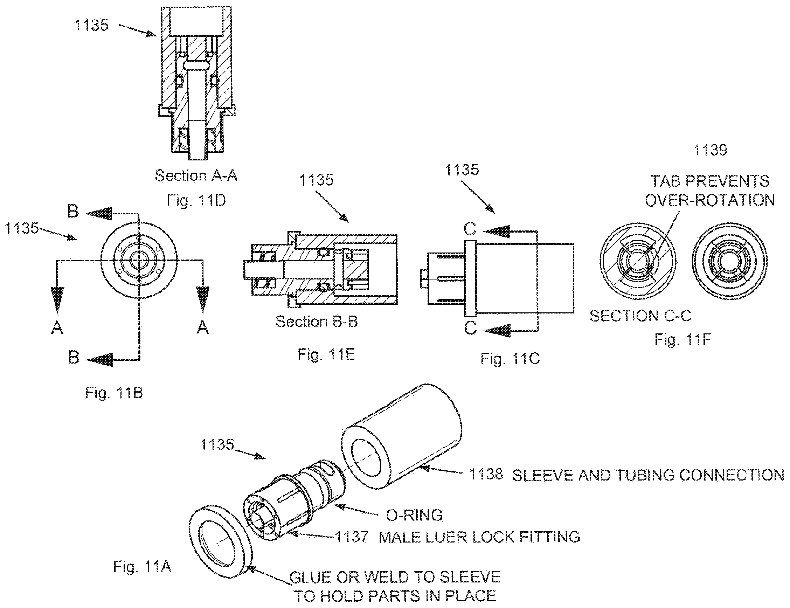

FIGS. 11A-11F disclose an embodiment of valves connecting the input and output tubing to trocars, such as valves 135, 235, 236, 935, and 936. Valve 1135 in FIG. 11A may include a rotatable collar with a movable section, such that when the valve 1135 is firmly connected the valve 1135 is open to allow gas flow and when disconnected prevents gas flow. FIG. 11A shows an exploded view of valve 1135, which may include a male luer lock fitting 1137 that joins with a female luer fitting (not shown) and rotates to allow gas flow. Valve 1135 may also include a sleeve and tubing connection 1138 to connect to input or output tubing, an O-ring to prevent leakage, and a part to hold the remaining components in place. FIG. 11B shows an end view of valve 1135 and FIG. 11C shows a side view of valve 1135. FIGS. 11D, 11E, and 11F show section views of valve 1135. FIG. 11F shows tabs 1139 that prevent over-rotation of the male luer lock 1137 fitting portion of valve 1135.

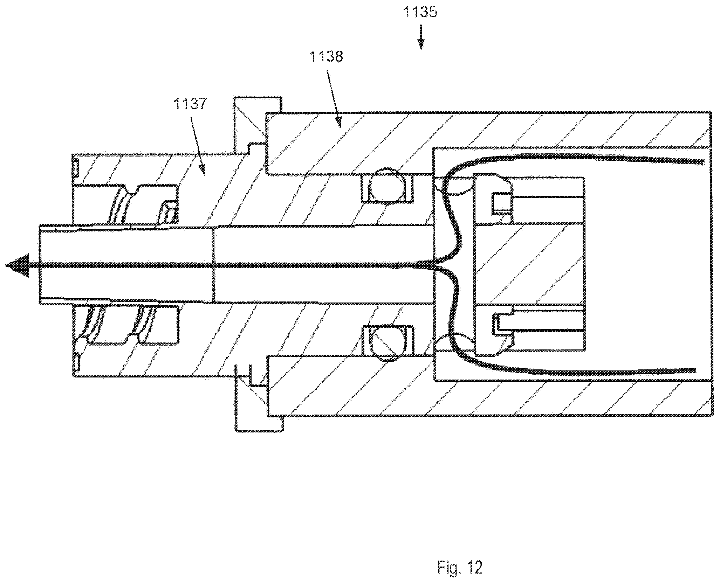

FIG. 12 discloses a sectional view of valve 1135 in the open flow configuration. The arrows in FIG. 12 disclose the gas flow path through valve 1135 when male luer lock fitting 1137 is rotated to allow gas flow. When valve 1135 is connected to a trocar, the male luer lock fitting 1137 rotates inside a stationary sleeve 1138, aligning openings in the male luer lock fitting 1137 with openings in the sleeve 1138 and allowing gas to pass through. When the valve 1135 is disconnected, the openings become misaligned and block the flow of gas.

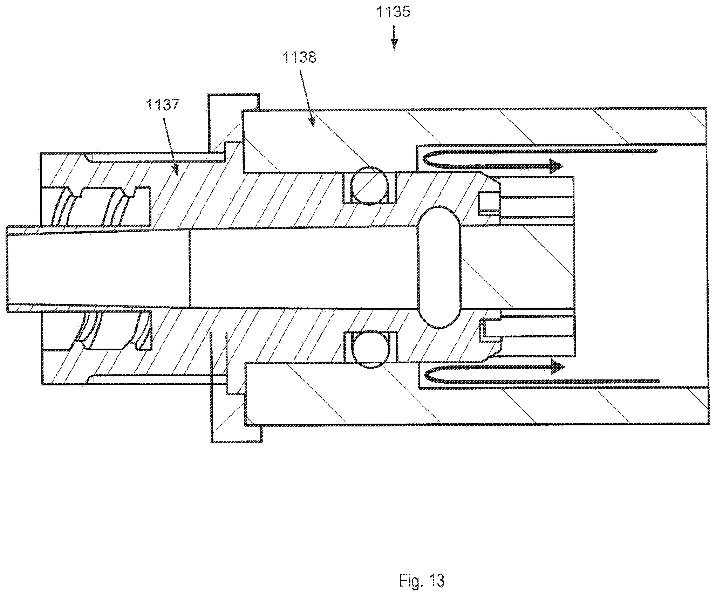

FIG. 13 discloses a sectional view of valve 1135 in the closed flow configuration. The arrows in FIG. 13 disclose the gas flow path stopping in valve 1135 when male luer lock fitting 1137 is rotated to prevent fluid flow.

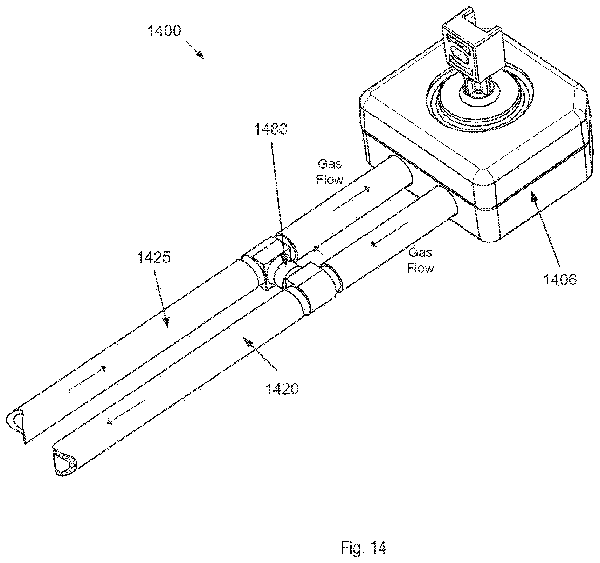

FIG. 14 discloses an embodiment of a gas recirculation system 1400. Gas recirculation system 1400 may include similar components and operating characteristics as the gas recirculation systems described in FIGS. 1-13. Gas recirculation system 1400 may include a bypass valve 1483 that is located between output tubing 1425 and input tubing 1420. Arrows located in FIG. 14 may show the gas flow paths. Bypass valve may be normally closed such that there is no gas flow path between output tubing 1425 and input tubing 1420. When bypass valve 1483 is open it may create a gas flow path from input tubing 1420 to output tubing 1425 as shown by the arrows in FIG. 14. The gas flow path from input tubing 1420 to output tubing 1425 may create a circulating gas loop around the pump cartridge 1406 that may limit the downstream pressure that can be generated during the pumping cycle. For example, opening bypass valve 1483 may divert a portion of the gas flow from the pump cartridge 1406 into the output tubing 1425, which may prevent a pressure increase downstream of bypass valve 1483. Bypass valve 1483 may be a one-way pressure relieve valve, such as a Minivalve or a Halkey/Roberts^{®} valve, such as a Duck Bill valve or a Spring Loaded valve. Bypass valve 1483 can be selected to open automatically based on the pressure present at the inlet side of bypass valve 1483, or at another location downstream of pump cartridge 1406. For example, bypass valve 1483 can be selected to open at a pressure as low as 0.1 psi to a pressure higher than 10 psi, depending on the application. It may be preferred that bypass valve 1483 open when the pressure is approximately in the range of 0.15 psi to 0.55 psi.

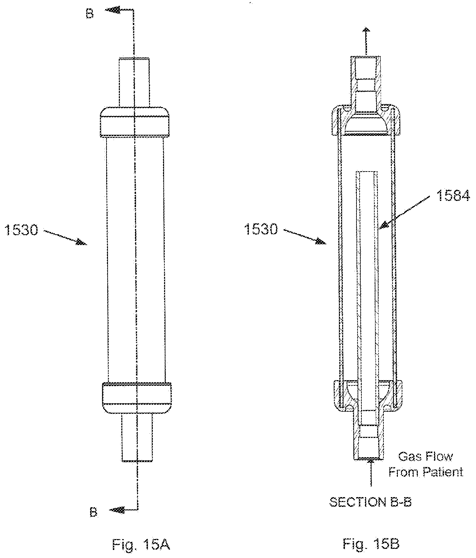

FIGS. 15A-B disclose an embodiment of a moisture trap, such as moisture traps 130, 230, and 930. Moisture trap 1530 in FIGS. 15A-B may be located in the output tubing (not shown) where gas flows from the patient to the recirculation pump (not shown), as shown by the arrows in FIG. 15B. FIG. 15B shows a section view of moisture trap 1530 that includes tube 1584 that extends within moisture trap 1530. Tube 1584 begins at the gas inlet side of moisture trap 1530 and may extend toward the outlet of moisture trap 1530, but may not contact the outlet of moisture trap 1530 such that there is a gap between the end of tube 1584 and the outlet of moisture trap 1530. The gap may allow liquid located within the gas to rain out before the gas reaches the outlet of moisture trap 1530. The liquid that is removed from the gas may collect within moisture trap 1530. The size of the gap between the end of tube 1584 and the outlet of moisture trap 1530 may be varied based on the application. For example, applications with higher gas velocities may require a larger gap to allow the liquid in the gas to rain out, whereas applications with relatively lower gas velocities may require a smaller gap to allow the liquid in the gas to rain out. Moisture trap 1530 may not include absorbent media to collect the liquid within moisture trap 1530. Moisture trap 1530, and its components, may be constructed of any suitable material to be in contact with liquid, such as plastic or metal.

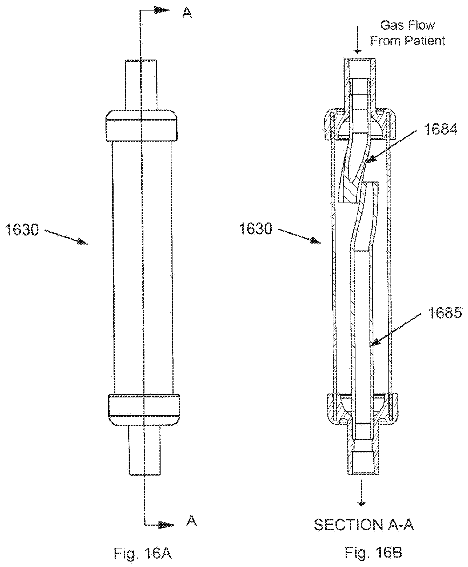

FIGS. 16A-B disclose another embodiment of a moisture trap, such as moisture traps 130, 230, 930, and 1530. Moisture trap 1630 in FIGS. 16A-B may be located in the input tubing (not shown) where gas flows from the recirculation pump (not shown) to the patient, as shown by the arrows in FIG. 16B. FIG. 16B shows a section view of moisture trap 1630 that includes input tube 1685 and output tube 1685 that both extend within moisture trap 1630. Input tube 1684 begins at the gas inlet side of moisture trap 1630 and may extend toward the outlet of moisture trap 1630. Output tube 1685 begins at the gas outlet side of moisture trap 1630 and may extend toward the inlet of moisture trap 1630. Input tube 1684 and output tube 1685 may extend past each other within moisture trap 1630, creating an overlap as shown in FIG. 16B, such that gas entering moisture trap 1630 from inlet tube 1684 cannot flow directly into output tube 1685 without first flowing through the interior of moisture trap 1630. FIG. 16B shows that inlet tube 1684 and outlet tube 1685 may include bends such that portions of the tubes overlap while the inlet of inlet tube 1684 and the outlet of outlet tube 1685 remain axially aligned. Liquid within the gas may rain out while it is flowing through the interior of moisture trap 1630 and before it flows out of moisture trap 1630 through output tube 1685. The liquid that is removed from the gas may collect within moisture trap 1630. Moisture trap 1630 may not include absorbent media to collect the liquid within moisture trap 1630. Moisture trap 1630, and its components, may be constructed of any suitable material to be in contact with liquid, such as plastic or metal.

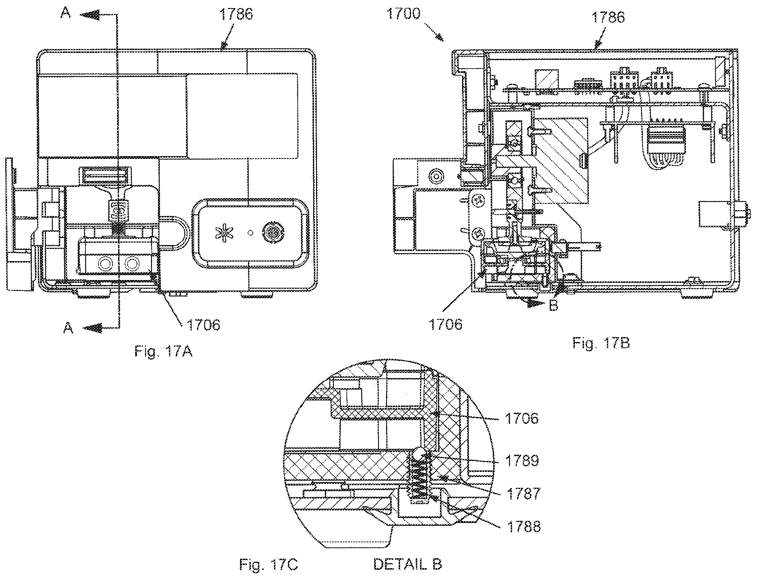

FIGS. 17A-C disclose an embodiment a gas recirculation system 1700. Gas recirculation system 1700 may include similar components and operating characteristics as the gas recirculation systems described in FIGS. 1-16. Gas recirculation system 1700 may include recirculation pump enclosure 1786 that houses some of the components of gas recirculation system 1700, such as a recirculation pump 1705, pump cartridge 1706, motor 1707, controller 1740, user interface 1745, power supply 1776, DC motor control circuitry 1778, and processor circuitry 1780.

FIG. 17A discloses gas recirculation system 1700 with the cartridge door open showing pump cartridge 1706 installed inside enclosure 1786. FIG. 17C is a detailed section view of gas recirculation system 1700 showing the pump cartridge locking mechanism 1787. Pump cartridge locking mechanism 1787 may include spring 1788 with ball 1789 located at one end of spring 1788. Spring 1788 may exert force on pump cartridge 1706 through ball 1789, which may lock pump cartridge 1706 within enclosure 1786. Alternatively, spring 1788 may exert force directly on pump cartridge 1706 without ball 1789.

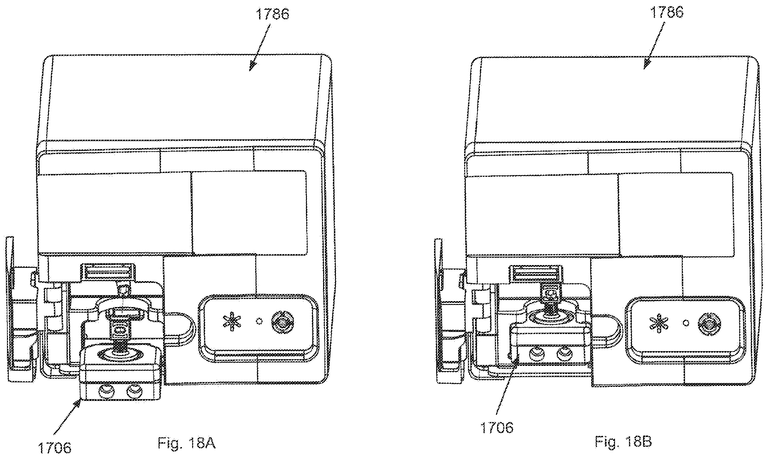

FIG. 18A is a perspective view of gas recirculation system 1700 with pump cartridge 1706 in a pre-insertion position. FIG. 18B is a perspective view of gas recirculation system 1700 with pump cartridge 1706 installed in enclosure 1786.

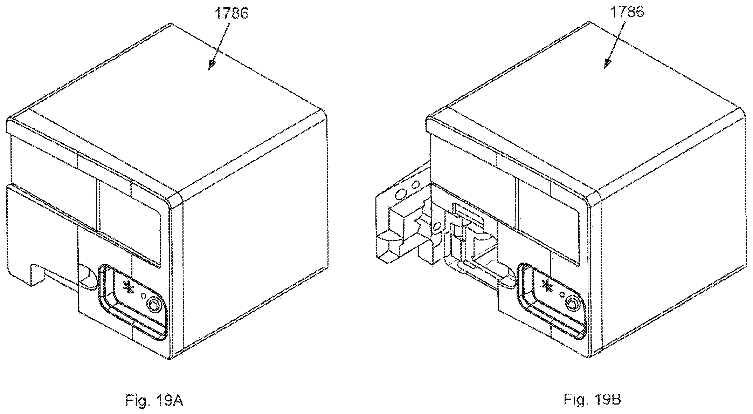

FIG. 19A is a perspective view of gas recirculation system 1700 with the cartridge door closed. FIG. 19B is a perspective view of gas recirculation system 1700 with the cartridge door open and without pump cartridge 1706.

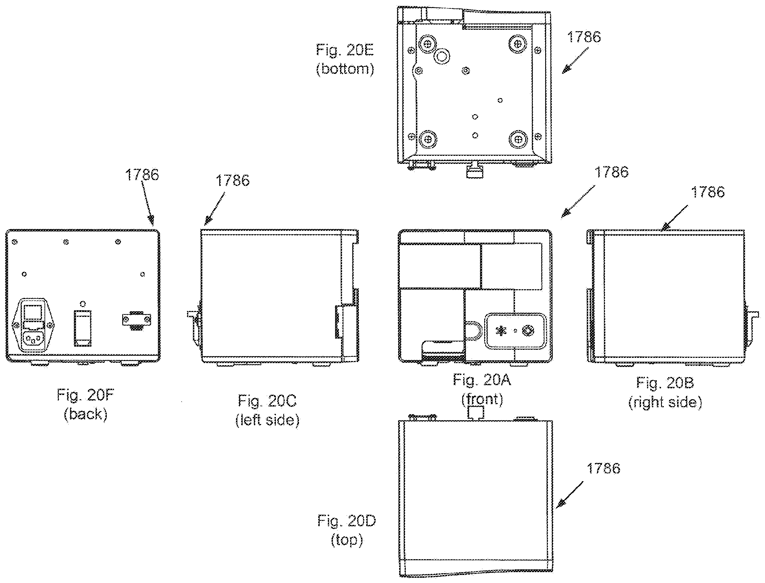

FIGS. 20A-20F disclose views of gas recirculation system 1700 with the cartridge door closed. The dimensions shown in FIGS. 20 A-20F are exemplary and may be modified based on the application of gas recirculation system 1700.

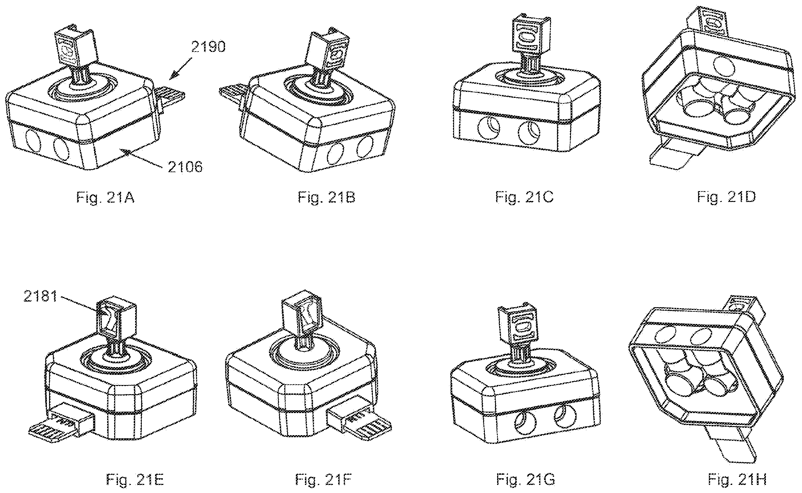

FIGS. 21A-21H disclose an embodiment a gas recirculation system 2100. Gas recirculation system 2100 may include similar components and operating characteristics as the gas recirculation systems described in FIGS. 1-20. Gas recirculation system 2100 may include pump cartridge 2106 with a coded connector 2190. Connector 2190 may be described in U.S. Patent No. 9283334. Connector 2190 may be able to identify if the correct pump cartridge 2106 is connected to recirculation pump 2105, if pump cartridge 2106 has been used previously, or to select and set gas recirculation system 2100 to operate according to special settings, such as flow rates. FIGS. 21A - 21H show perspective views of pump cartridge 2106 with connector 2190.

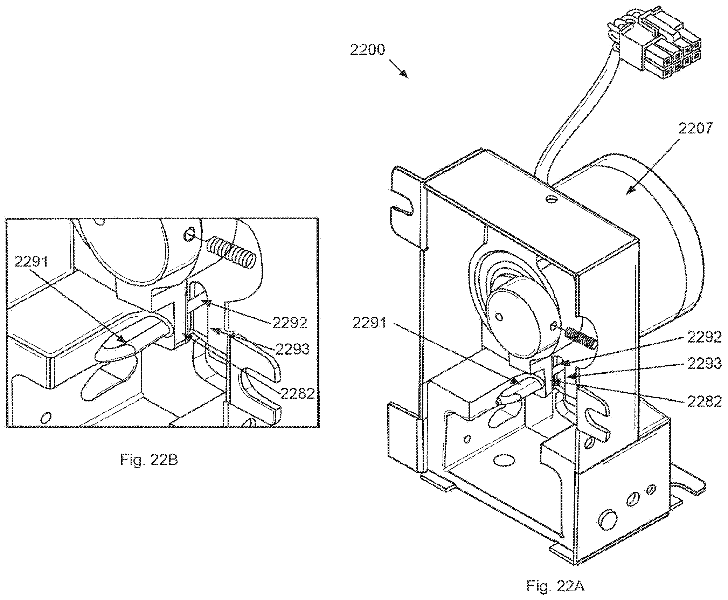

FIGS. 22A-B disclose an embodiment a gas recirculation system 2200. Gas recirculation system 2200 may include similar components and operating characteristics as the gas recirculation systems described in FIGS. 1-21. Gas recirculation system 2200 may include components that allow the pump cartridge 2206 (not shown) to be coupled with the motor coupling arm 2282 in a "blind" manner, such that a user may insert the pump cartridge 2206 into the gas recirculation system enclosure 2286 (not shown) without knowing the exact location of the motor coupling arm 2282 and without bending over to look inside enclosure 2286 to see the location of the motor coupling arm 2282. FIG. 22A shows a coupling shaft 2291 extending from the front of the motor coupling arm 2282. Coupling shaft 2291 may include a tapered end portion to aid insertion of the coupling shaft 2291 into the corresponding opening in diaphragm actuator 2281 (not shown in FIGS. 22A-B, shown in FIGS. 5, 6, 8, and 21). Diaphragm actuator 2281 may include a corresponding tapered opening (as shown in FIG. 21). A locating pin 2292 may extend from the back of the motor coupling arm 2282. Locating pin 2292 may fit within locating slot 2293. FIG. 22B shows a detail view of the coupling shaft 2291 and locating pin 2292 extending from the motor coupling arm 2282. Locating slot 2293 may be found in the mount for motor 2207 or other stationary portion of enclosure 2286. Locating pin 2292 will move up and down in locating slot 2293 as motor 2207 causes motor coupling arm 2282 to move up and down. Locating slot 2293 will restrict the side to side motion of locating pin 2292. Because locating pin 2292 is connected with motor coupling arm 2282, the restricted side to side motion of locating pin 2292 will ensure that motor coupling arm 2282 and coupling shaft 2291 remain in approximately the same vertical plane regardless of where motor coupling arm 2282 is located when motor 2207 stops. Accordingly, a user may easily insert pump cartridge 2206 into enclosure 2286 and couple diaphragm actuator 2281 with motor coupling arm 2282.

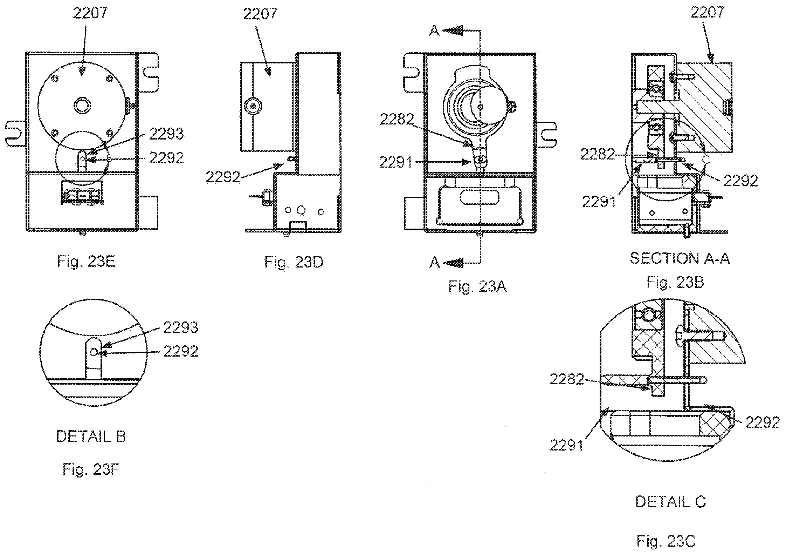

FIGS. 23A-F disclose views of portions of gas recirculation system 2200. FIG. 23A shows a front view of the mount for motor 2207 and motor coupling arm 2282 with coupling shaft 2291. FIG. 23B shows a section side view of the mount for motor 2207 along with motor 2207, coupling shaft 2291, and locating pin 2292. FIG. 23C shows a detail view of coupling shaft 2291 and locating pin 2292 found on the front and back, respectively, of motor coupling arm 2282. FIG. 23D shows a side view of the mount for motor 2207 along with motor 2207 and locating pin 2292 as it extends through locating slot 2293. FIG. 23E shows a back view of the mount for motor 2207 along with motor 2207 and locating pin 2292 as it extends through locating slot 2293. FIG. 23F shows a detail back view of locating pin 2292 as it extends through locating slot 2293.

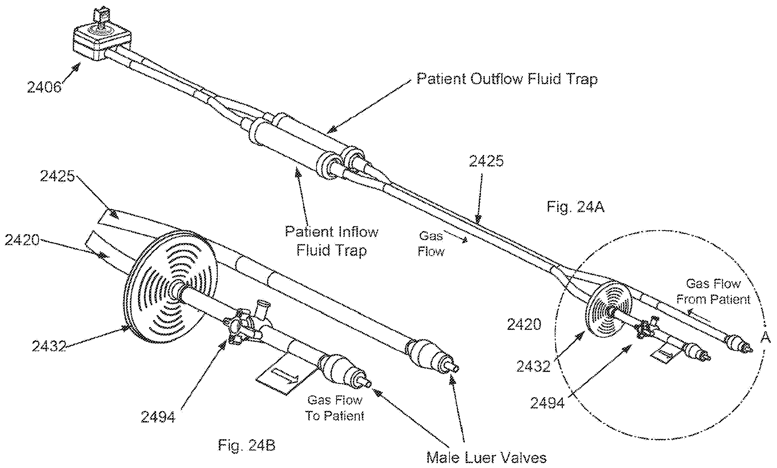

FIGS. 24A-B disclose an embodiment a gas recirculation system 2400. Gas recirculation system 2400 may include similar components and operating characteristics as the gas recirculation systems described in FIGS. 1-23. Gas recirculation system 2400 may include components to evacuate CO₂ gas from a patient's peritoneal cavity after laparoscopic surgery is completed. Typically, when laparoscopic surgery is completed, a luer connection on a trocar that inserted into the patient is opened, which allows CO₂ gas from within a patient's peritoneal cavity to escape into the operating room. Undesirably, the escaping CO₂ gas is not filtered and may contain aerosolized chemicals, particles, bacteria, etc. that remains from the operative procedure.

Gas recirculation system 2400 may include three-way valve 2494 located in input tubing 2420. Input tubing 2420 flows to the patient. FIG. 24A shows that three-way valve 2494 may be located downstream of filter 2432 so that any gas flowing through three-way valve 2494 has already had impurities filtered out. FIG. 24B is a detail view of gas recirculation system 2400 showing three-way valve 2494, input tubing 2420, output tubing 2425, and filter 2432. At the end of a surgical procedure, before the recirculation tubing 2420, 2425 is removed and while the recirculation pump 2405 is still operating, three-way valve 2494 may be configured to prevent gas flow to the patient and to allow gas flow to the operating room. In this manner, the recirculation pump 2405 will pump out the CO₂ gas from within the patient's peritoneal cavity with filter 2432 preventing any contamination from leaving the patient. Utilizing three-way valve 2494 to allow gas flow to the operating room, rather than simply disconnecting input tubing 2420 from the patient, ensures that the only gas from the patient entering the operating room is filtered through filter 2432 first by maintaining all the gas connections with the patient that existed during the surgical procedure. Utilizing three-way valve 2494 to remove the CO₂ gas from the patient may reduce the risk to operating room staff without requiring an additional means for insuring the cleanliness of the escaping CO₂ gas.

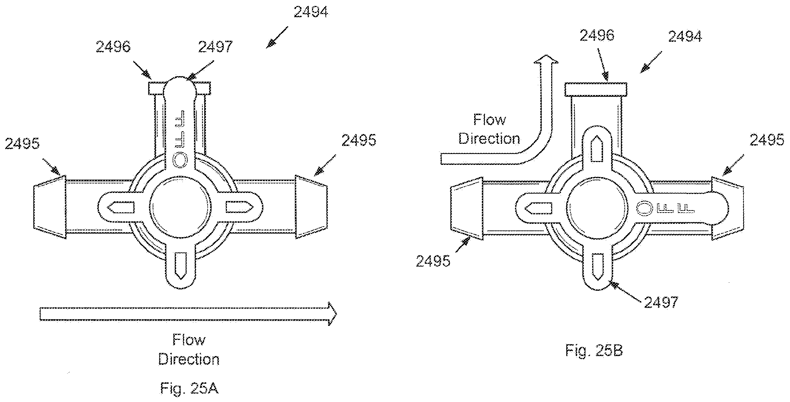

FIGS. 25A-B show three-way valve 2494 isolated from input tubing 2420. Three-way valve 2494 may include two in-line barbed fittings 2495 to connect with input tubing 2420. Three-way valve 2494 may also include a female luer connection 2496 oriented perpendicularly to the two in-line barbed fittings 2495. The female luer connection 2496 may be used for pressure relief purposes, such as to release CO₂ gas into the operating room. Three-way valve 2494 may also include a stopcock 2497 that rotates to adjust the open flow path of three-way valve 2494. As shown in FIG. 25, the closed flow path through three-way valve 2494 is indicated by the "OFF" portion of stopcock 2497. FIG. 25A shows three-way valve 2494 configured to allow gas flow through the two in-line barbed fittings 2495, which may be connected with input tubing 2420 leading to the patient. FIG. 25A may be the configuration used during recirculation function. The configuration of three-way valve 2494 in FIG. 25A may prevent gas from being released into the operating room. FIG. 25B shows three-way valve 2494 configured to allow gas flow out through the female luer connection 2496 and into the operating room. FIG. 25B may be the configuration used at the end of the surgical procedure when gas is being evacuated from the patient. The configuration of three-way valve 2494 in FIG. 25B may prevent gas flowing to the patient.

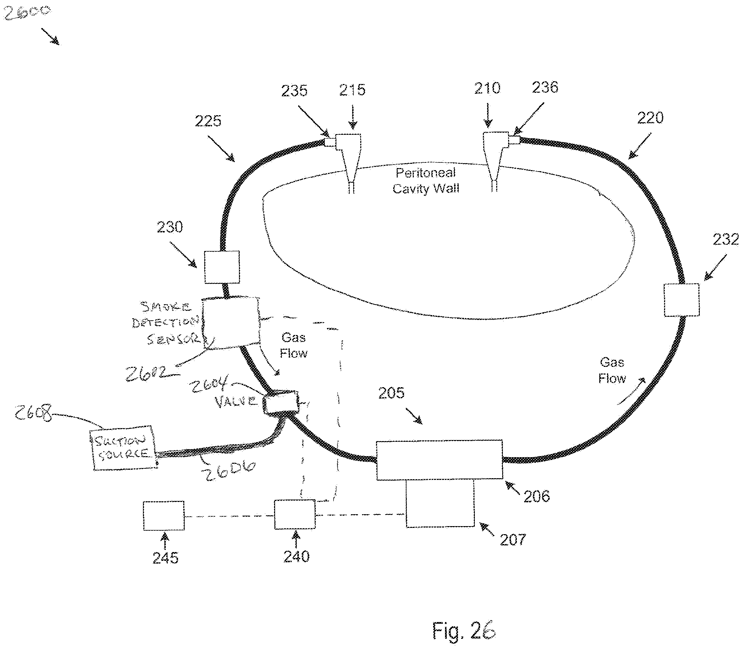

Another version of a gas recirculation system including automatic responsiveness to changes in detected smoke content, and which may include any one or more of the features of the gas recirculation systems of 1-25 above, is now described. The example gas recirculation system 2600 of FIG. 26 with smoke detection includes many of the same features and components described in FIG. 2 for gas recirculation system 200. In FIG. 26, like components from FIG. 2 are labeled with the same identifying numbers as in FIG. 2. Unlike gas recirculation system 200, however, gas recirculation system 2600 also includes a smoke detection sensor 2602 in communication with the controller 240. Also, as noted in FIG. 26, a valve 2604 is included that is controllable by the controller 240 to either route gas from the tubing exiting the peritoneum to the recirculating pump 205, or to a suction exhaust tube 2606 that receives suction from a suction source 2608 and vents the gas and smoke to a destination outside of the closed system. As discussed in different implementations below, the valve 2604, suction exhaust tube 2606 and suction source 2608 may be omitted in some embodiments.

The gas recirculation system 2600 may include a smoke detection mechanism that automatically starts and stops, or automatically adjusts the rate of, filtering of smoke generated by a procedure. The smoke detection sensor 2602, which may be positioned in the gas pump 205, along the gas pathway between the gas recirculation device and the peritoneum (as shown), or even in the peritoneum itself, is in communication with the controller 240. In order to provide a controlled environment for measuring the smoke, it is expected that the smoke detection sensor 2602 may be implemented as a standalone sensor along the path of the gas tubing exiting the peritoneum, rather than in the peritoneum or in the SRS itself, however these other locations are also contemplated. The controller 240 may use the information on the amount, or the amount and duration, of smoke detected by the sensor 2602, to automatically adjust the speed of the motor 207 of the gas recirculation motor in the pump 205 based on the determined amount and/or duration of smoke detected. The gas recirculation system 2600 may also optionally include a valve 2604 that is selectively controllable by the controller 240 to choose between recirculating the gas within the closed system of input and output tubing 220, 225 and the gas removal filters 230, 232 included therein, and a suction exhaust tube 2606 that connects with a suction source 2608 that will quickly remove the gas and smoke if the smoke density rises above a predetermined maximum. Any of a number of known suction sources, including standalone pumps or built-in wall-mounted suction ports in medical facilities, may be used as the suction source 2608.

The suction source 2608 may be fixed at a predetermined flow rate or adjustable to any of a number of flow rates. In one implementation, although a flow rate in the range of 10-12 liters per minute may be acceptable for the suction source 2608, it is contemplated that the suction source 2608 may be capable of a minimum flow rate of 12 liters per minute (lpm). In some implementations, a minimum flow rate of 15 lpm for the suction source is contemplated to provide a faster flow than supported by the motor when the smoke accumulation is too fast for the pump to handle on its own. In implementations when the suction source is a fixed flow rate source, the suction source may be always on, or controlled to be at either an on or off state by the controller 240. The valve 2604 may be adjusted by the controller 240 to adjust the suction flow rate applied to the recirculating gas being removed in implementations where the suction source 2608 is either always on or is only controllable between on and off states. In other implementations, the suction source 2608 may be a variable flow rate suction source adjustable by the controller 240.

Different types of smoke detector technologies are contemplated for the smoke detector sensor 2602. Examples of these different technologies may include electrochemical sensors, chromatographic, optical or photoelectric sensors, audio sensors or electrical pairing. Suitable electrochemical sensors include ionizing smoke detector circuits, for example ones that use Americium 241 to ionize gas and generate a current in adjacent electrodes. Any of a number of other electrochemical gas sensors, such as the Figaro TGS5342 or TGS5141-P00 electrochemical sensors available from Figaro USA Inc. of Arlington Heights, IL, may also be utilized. With respect to chromatographic or photoelectric sensors, optical or photoelectric smoke detectors that sense direct or reflected light from a light source such as a light emitting diode (LED) may be implemented in different embodiments. Audio detectors may be implemented to detect the sound of a smoke generating device (e.g. a cautery device) in operation to trigger the smoke evacuation functions described herein. Also, a direct electrical pairing between the smoke generating device and the smoke evacuation equipment may be used so that operation of the smoke generating device causes a signal to be transmitted to the smoke evacuation system to stop and start in coordination with use of the cautery or other smoke generation device by a surgeon. Only one sensor technology may be implemented as the smoke sensor in one embodiment. In other embodiments, a combination of more than one of the sensor technologies may be implemented.

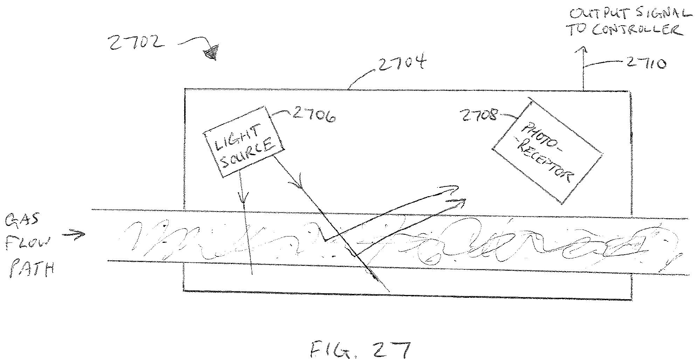

One example of a smoke detection sensor using a photoelectric sensor arrangement is shown in FIG. 27. In the embodiment of FIG. 27, the sensor 2702 is positioned in-line along the gas flow path, such as adjacent to, on or in the tubing 2703 carrying the gas from the peritoneum. The sensor includes a housing 2704 that includes a light source 2706, such as an infrared laser or bright LED, that emits light across the gas flow path. A photoreceptor 2708 positioned within close proximity to the light source 2706 may be placed in a dispersion detection arrangement so that no light reaches the photoreceptor 2708 in a direct path from the light source 2706. Instead, the photoreceptor 2708 is positioned at an angle which can only receive light from the light source 2706 that has been reflected from the gas after leaving the light source. Thus, when smoke enters the gas flow path, the light from the light source 2706 is reflected by the particulate matter in the smoke to the photoreceptor 2708, causing the photoreceptor to generate an output signal that is communicated to the controller 240. The smoke detector sensor may transmit this output signal over a wired or a wireless connection 2710 to the controller 240. In different embodiments, the output signal can be improved with more using more than one photoreceptor 2708 in the smoke detection sensor 2702 to capture more of the dispersed of light.

In alternative implementations, the infrared light source 2706 and the photoreceptor 2708 may be oriented to directly face one another such that a direct beam of infrared light from a light source reaches the photoreceptor. In this direct beam arrangement, any smoke in the gas stream would reflect some or all of the light such that less light reaches the photoreceptor 2708. The output signal magnitude may be used by the controller 240 to determine the amount of smoke present in the gas. In some implementations, the circuitry in the sensor 2702 may be configured such that the magnitude of the output signal increases as smoke density increases. Alternatively, an amount of reduction of magnitude of a known initial output signal level may indicate the amount of smoke present in the gas flow.

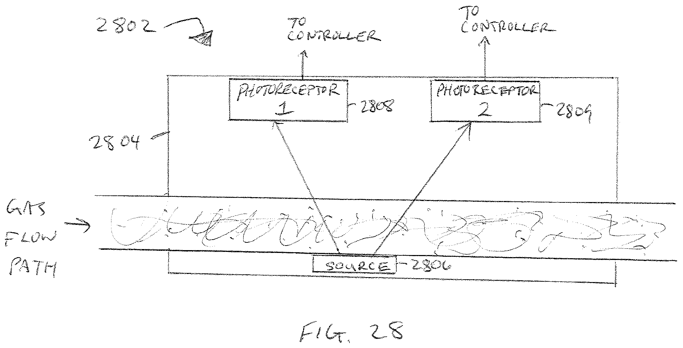

In yet another embodiment of a chromatographic/photoelectric sensor arrangement, as illustrated in FIG. 28, the smoke detection sensor 2802 may be a dual wavelength/dual detector sensor based on non-dispersive infrared (NDIR) principles. This alternative type of sensor 2802 may also be suitable for placement along the gas flow path and include a housing 2804 that has two photoreceptors 2808, 2809 in the direct path of a strong, broadband light source 2806, which may also be referred to herein as an emitter, that generates the wavelengths necessary for CO₂ detection. For example, in systems recirculating CO₂ gas, one photoreceptor 2808 may be set to detect a 4.2µm wavelength that is absorbed by CO₂ gas and a second photoreceptor 2809 may be set to detect a 3.9µm wavelength which is not affected by any gas, but would be affected by the particulate matter of any smoke. The combination of the outputs of the photoreceptors may then permit the controller 240 to determine the quality of the CO₂ gas in parts per million. In one alternative implementation, the photoreceptors 2808, 2809 may each include a different thin film filter (TFF) which has a wavelength bandpass profile tuned to detect a respective different one of the constituent gases or particulates in the smoke, such as carbon monoxide.

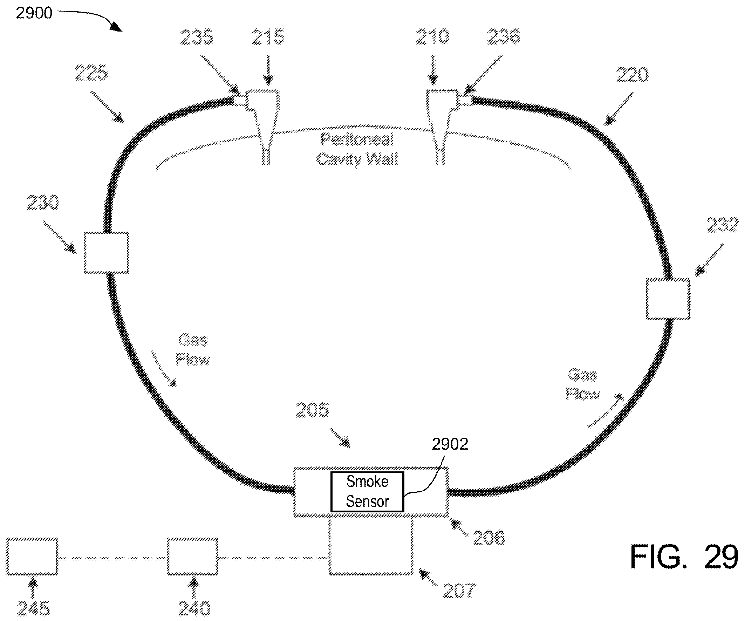

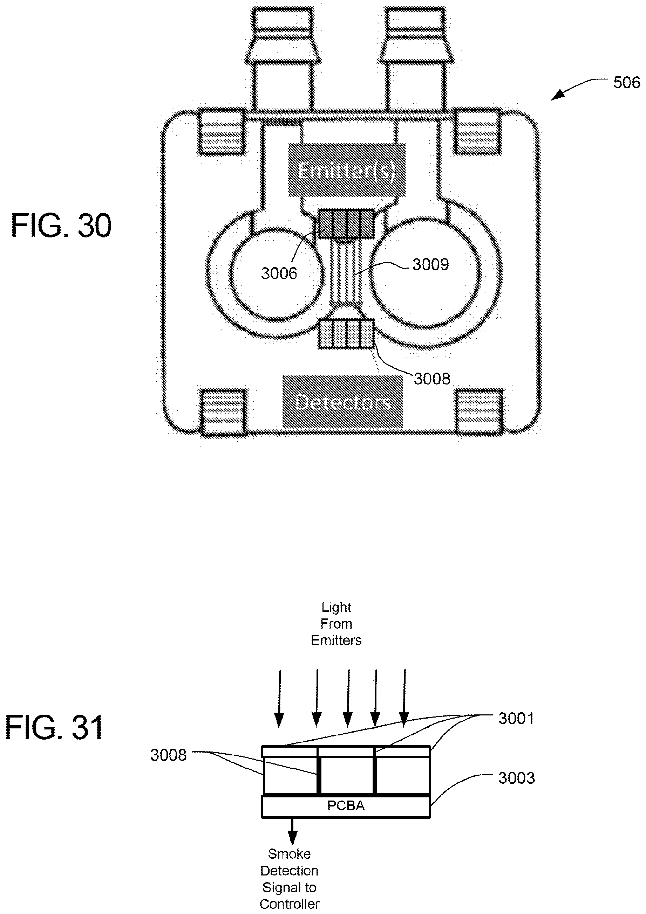

Referring to FIG. 29, the smoke sensor 2902 may be positioned in or adjacent to the pump 205, rather than in or adjacent the input or output tubing. In some embodiments utilizing one or more photoelectric emitters and one or more photoelectric detectors as the smoke sensing sensor technology, the sensor 2902 may be located in the pump 205, for example in the removable cartridge 206 of the pump 205. As illustrated in FIG. 30, one or more photoelectric emitters 3006 and multiple detectors 3008 may be positioned adjacent to the cartridge 506 such that circulation flow passes through the optical path(s) 3009 between the emitter(s) 3006 and the detectors 3008. The emitter(s) 3006 and detectors 3008 are not in fluid contact with the recirculating gas but are instead isolated by the cartridge material(s) which, at least in the region adjacent to the photoelectric emitters 3006 and detectors 3008, are transparent to the wavelengths transmitted by the photoelectric emitters. Physical isolation of the sensors from the gas may thus improve the system's bio-incompatibility risks versus fluid-contacting sensors. Additionally, this isolation may improve system robustness by protecting the sensors from degraded performance through corrosion and contamination with water condensation and other chemical constituents of surgical smoke. A modified version of cartridge 506 from FIG. 5 is shown in FIG. 30, but any of the previously illustrated pump cartridges may be modified to include the sensors disclosed herein.

Electrical contact for supplying power to the emitter(s) 3006 and detector(s) 3008 of the sensor may be made at the point where the cartridge is inserted into the recirculation device. Locating the detector(s) 3008 proximally to the recirculation pump reduces the signal transmission distance and the degree of electromagnetic signal interference. Locating the detectors 3008 proximally to the pump may also minimize the need for wires which could compromise flow or biocompatibility if passed within the tubing set. Additionally, locating the sensor at the cartridge may help avoid operating room clutter and tangling/tripping hazard that a longer wiring run passed outside the tubing set from a sensor located further from the pump along the tubing.

Referring to FIG. 31, an array of exemplary detectors 3008 of the smoke sensor 2902 are shown. The detectors of the sensor may each be equipped with a thin film filter (TFF) 3001, each of which has a wavelength bandpass profile tuned to detect a different one of the constituent gases or particulates in the smoke, such as described above. The respective signal levels from these detector(s) are synthesized by the recirculation pump controller, allowing various gas constituent signatures and concentrations to be detected. Power for powering the sensor 2902 may be obtained by a wired connection, for example by electrical contacts arranged on the removable cartridge 506 that mate with electrical contacts on the housing of the pump 205. The data signals from the detectors 3008 may be transmitted via wires to the controller 240, or wirelessly. For instance, instead of wires to communicate data to the controller or other parts of the smoke removal system, a printed circuit board assembly (PCBA) 3003 which may comprise an integrated circuit is electrically connected to the each detector 3008, or may be a series of integrated circuits each dedicated to a respective one of the detectors 3008. This PCBA may be configured to wirelessly transmit a signal back to the recirculation pump and/or pump controller to communicate the locally sensed output of the photoelectric sensor.

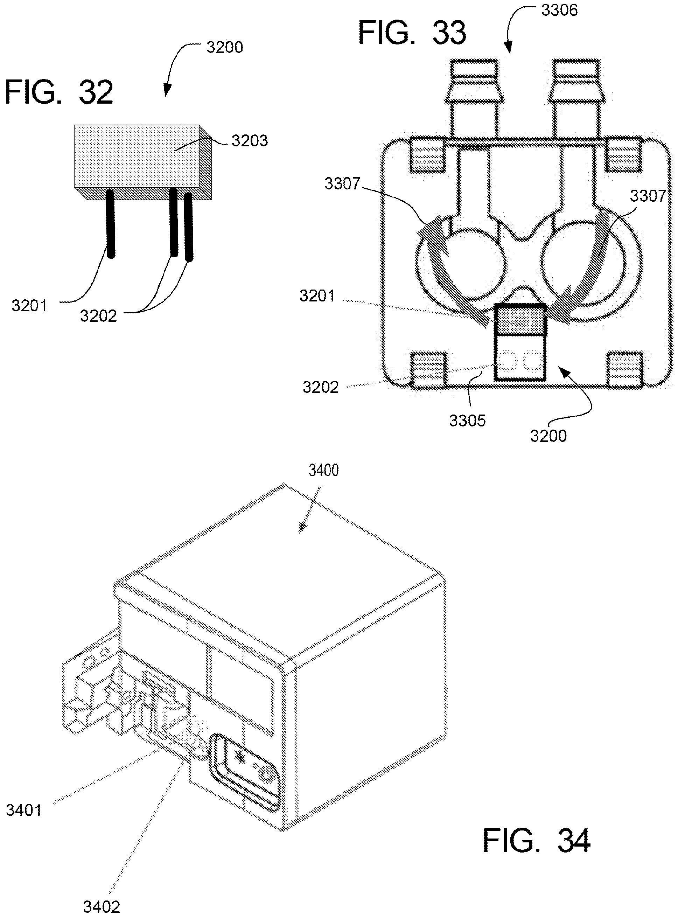

In other embodiments, the sensor 2602, 2902 may be an electrochemical sensor 3200 as shown in FIG. 32. As with the photoelectric sensor versions, the electrochemical sensor 3200 may be located along or in the tubing (output or input), or at the pump. The electrochemical sensor 3200 may have a working electrode 3201 and one or more counter electrodes 3202 in electrical communication with the electrochemical sensor circuitry 3203. In embodiments where the electrochemical sensor 3200, or at least the electrodes 3201, 3202 of the electrochemical sensor is placed in the input tubing, the working electrode 3201 of the electrochemical sensor is in fluid contact with the recirculating gas and wires may be used to make electrical connection to the controller 240 and remainder of the smoke recirculation device. In alternative embodiments, a wireless arrangement of the electrochemical sensor may be implemented instead of a wired version. For instance, instead of wires to communicate data to the controller or other parts of the smoke removal system, a wireless communication circuit may be integrated with the electrochemical sensor. Similar to the photoelectric sensor version describe above, the wireless communication circuit for the electrochemical sensor version may be an integrated circuit, or a discrete circuit on printed circuit board assembly, with an embedded wireless transmitter that is electrically connected to the electrochemical sensor. The wireless transmitter and may be configured to wirelessly transmit a signal back to a receiver on the recirculation pump and/or pump controller to communicate the locally sensed output of the electrochemical sensor 3200. The wired version or the wireless version of the electrochemical sensor embodiment may be placed along or in the input tubing, or the output tubing, or along or in the pump cartridge of the pump in different embodiments. For example, the electrochemical sensor 3200 may be located in a wall of the pump cartridge, such as a wall of cartridge 506 (FIG. 5B), where the working electrode 3201 is in fluid contact with the recirculating gas at the proximal portion of the tubing set. The electrochemical sensor 3200 may then makes electrical contact with the pump or other portion of the gas recirculation apparatus at the location of insertion.

As shown in FIG. 33-35, the electrochemical sensor 3200 may be placed in the bottom wall 3305 of the gas recirculation pump cartridge 3306. The electrochemical sensor 3200 may be oriented with the working electrode 3201 toward the inner, gas-contacting portion of the cartridge 3306, so that it is near the gas flow (shown with arrows 3307). The working electrode 3201 may be in fluid contact with the recirculating gas at the proximal portion of the tubing set and the counter electrodes 3202 (two electrical leads of) of the sensor exposed so that electrical connection is made with the corresponding mating pads in the pump, upon insertion of the pump cartridge 3306 into the pump.

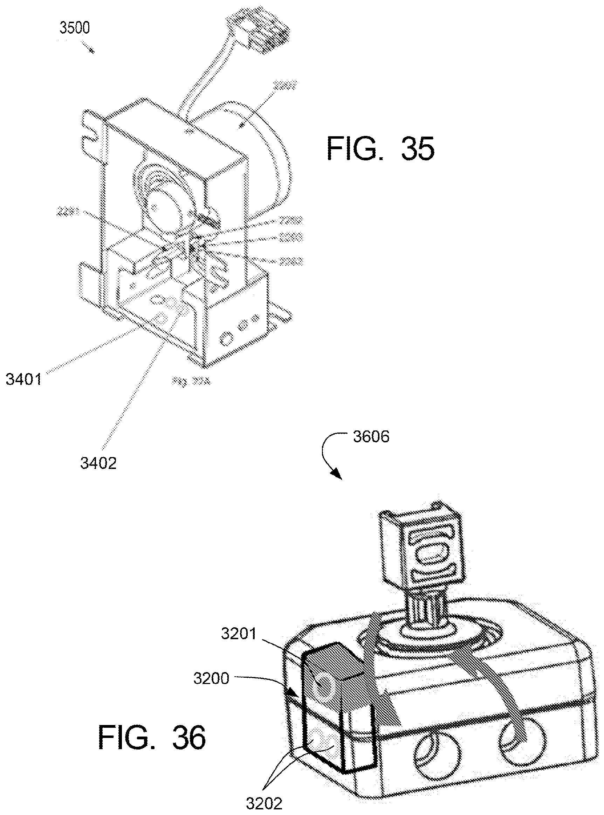

FIG. 34 illustrates a motor enclosure 3400 with modified motor assembly 3500 of the pump motor of the gas recirculation system, and FIG. 35 shows the modified motor assembly 3500 of the gas recirculation system removed from the motor enclosure 3400. The motor enclosure 3400 and modified motor assembly 3500 in FIG. 34-35 connect with pump cartridge 3306, but are shown without the pump cartridge 3306 to better illustrate the location of electrically conductive mating pads 3401, 3402 corresponding to the electrodes 3201, 3202 on the bottom wall of the pump cartridge 3306. The pump cartridge 3306 would then be removable insertable into the motor enclosure and motor assembly in the same manner as the cartridge 1706 is shown as inserted in FIG. 18A, above. Although the modified pump enclosure 3400 and modified motor assembly 3500 are shown as modified versions of the pump and motor systems shown in FIGS. 19B and 22A above, any of a number of different pump housing and recirculation system configurations that may work with a removable pump cartridge are contemplated. Also, as seen in the example of FIG. 36, other locations for the electrochemical sensor 3200 on a cartridge 3606, for example on a side wall of a cartridge 3606 rather than on the bottom as shown in FIG. 33, are contemplated. The complimentary mating pads in the motor enclosure and/or motor assembly would then be relocated to the corresponding side wall in those components.

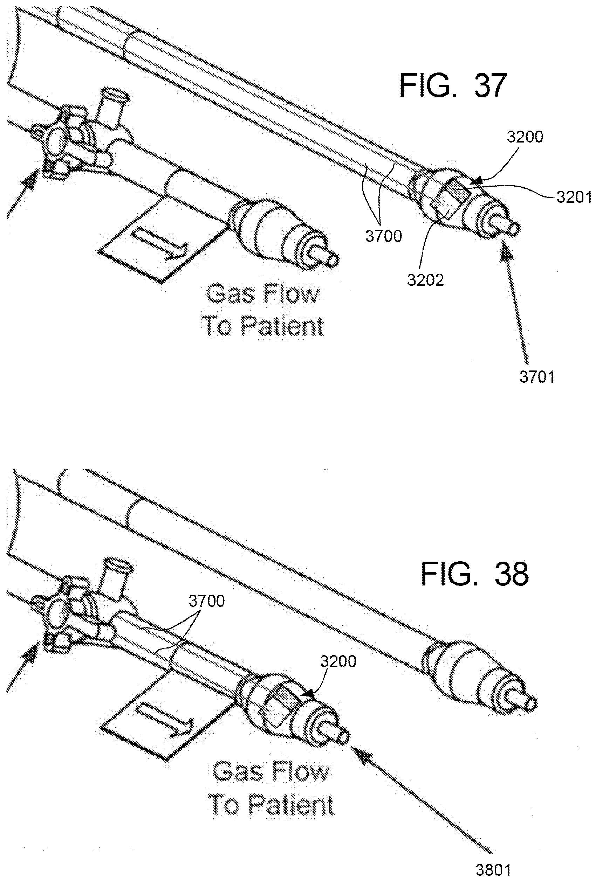

In yet other implementations, as shown in FIGS. 37-38, a smoke detection sensor such as electrochemical sensor 3200 may be placed in the input male Luer valve 3701 of the gas recirculation pump tubing set. In this manner, the working electrode may be in fluid contact with the recirculating gas at the proximal portion of the tubing set. Wires 3700 running from the electrochemical sensor 3200 to the recirculation pump may be used used to electrically transmit the signal from the sensor 3200 to the pump. FIG. 37 illustrates the sensor 3200 in the input Luer valve 3701 receiving gas from the patient, while FIG. 38 shows the sensor 3200 in the output Luer valve 3801 for the gas line to the patient. One or both Luer valves may house the electrochemical sensor 3200, or other type of smoke detection sensor, in different embodiments. Also, rather than wires 3600 running inside a tube from the sensor 3200 to the pump, wires 3600 could exit the tube set at or near the Luer valve and run outside the tube to the pump, pump controller or other destination. Placement of the sensor in a Luer connection may have certain advantages over other locations in the smoke removal system, including reduced response time and/or increased sensitivity due to closer proximity to the cauterization location. Additionally, relative to placement in the pump, this placement may extend sensor life and reduce possible wear by physically isolating it from the pump's mechanical oscillations.

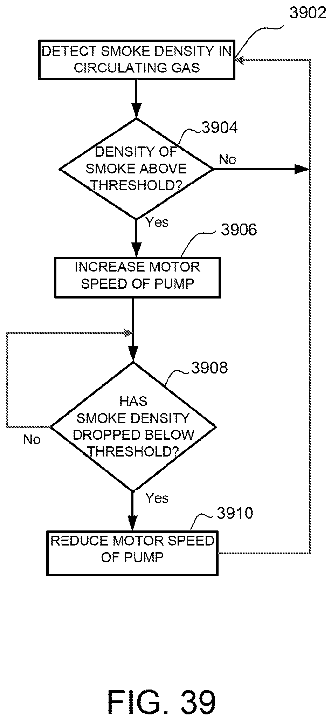

Utilizing the gas recirculation system of 26-38, methods of operation of the gas recirculation system 2600, 2900 are described below. In a first implementation, as illustrated in FIG. 39, the controller 240 of the gas recirculation system may monitor the output signal of the smoke detection sensor 2602, 2902 to detect the smoke density in the gas in the output tube 225 (at 3902). If the gas density detected is greater than a predetermined threshold (at 3904) then the controller 240 may communicate with the motor 207 of the pump 205 to increase the circulation speed of the gas and thus increase the speed at which smoke is filtered from the gas (at 3906). In one embodiment, where the smoke detection sensor is a light-based sensor (e.g., for detecting light from a light source such as a LED or laser source) the predetermined threshold may actually be a plurality of thresholds each associated with a different motor speed change based on the amount of light attenuation or reflection due to smoke in the gas. For example, the motor 207 of pump 205 may be set a default speed that increases, or decreases back toward the default speed if already increased, based on the light attenuation/reflection detected.

After increasing the speed of the gas circulation, the controller 240 may then check the sensor 2602, 2902 to see if the smoke density has decreased below the same (or a different) threshold density (at 3908). The controller may continue to check for a drop in the smoke density until it has dropped below the predetermined threshold and then reduce the motor speed (at 3910). This process may repeat. The timing of when the controller 240 checks the sensor 2602, 2902 and compares the density detected to the appropriate threshold may be continuous or may be at predetermined regular time intervals. Also, in one embodiment, the pump may be turned on or off in response to detected smoke density. Alternatively, the pump 205 may be configured for continuous operation and the controller increases motor speed from a default speed to higher speed, and then from that higher speed back down to the default speed. In alternative embodiments, the motor 207 is capable of variable adjustments in speed beyond an on or off setting or between an initial non-zero speed and a higher speed setting. In this alternative embodiment, the controller 240 is configured to vary the speed of the motor in direct proportion to the density of smoke detected (e.g. the magnitude of the output signal received from the sensor 2602, 2902) between a minimum speed and a maximum speed in steps only limited by the granularity of speed adjustment for the motor 207. The motor may be an infinitely variable speed motor or have a plurality of different speeds that the controller can access.

In one implementation, the relation of pump motor speed increase to sensed smoke in the gas being recirculated may be based on an amount of light attenuation detected in the sensor 2702, 2802. For example, starting from a default pump motor speed, the controller may not make any change to motor speed if the attenuation detected of the light projected into the gas is 1% or less. Pump motor speed may be increased by 10% if the attenuation detected is greater than 1% and less than or equal to 3%, and increased by 25% if the attenuation detected is greater than 3% and less than or equal to 10%. Finally, in this implementation the motor speed may be increased by 40% if the attenuation of the light is detected at greater than 10%. One default flow rate that may be utilized (when less than or equal to 1% light attenuation detected) may be 12 liters per minute (lpm) for the gas recirculation system and the increased pump motor speeds based on detected smoke increases the rate from there. It is expected that the relation between increase in pump motor speed and increase in flow rate will not be linear, however controller 240 may be set to increase motor speed by a desired percentage or may compensate to adjust motor speed to achieve the desired flow rate increase in other implementations. In another implementation. the default motor speed or flow rate may be user adjusted and the ranges of increased motor speed or flow rate may be user defined via the controller.

More sophisticated smoke sensing criteria, beyond that of instantaneous smoke density as described above, may be implemented by the controller 240 in different embodiments. For example, the on/off or low speed/higher speed decision of FIG. 39 executed by the controller 240 may be combined with a duration factor, such as an elapsed time or the number of consecutive discrete measurements made where the detected smoke density is above or below the relevant predetermined threshold. Thus, the method of FIG. 29, may be modified in steps 3904 and 3908 to require that the (for step 3904) smoke detected has been above a predetermined level for a predetermined time before triggering an increase in motor speed. Similarly, for step 3908, the controller may be configured to reduce the motor speed only when the combination of smoke density and time duration meet the predetermined lower threshold. The two-factor density and time analysis may also lead to a range of different combinations - for example either a very high smoke density for a shorter duration, or an slightly lower smoke density over a longer duration, may both result in the controller increasing the speed of the motor. The controller may be programmed to execute code stored in its memory, or a separate memory, to process the sensor output from the smoke detection sensor with an internal clock signal or counter to determine whether the predetermined threshold requirements for raising or lowering the motor speed of the pump 205 have been met. Similarly, as discussed with the smoke density-only embodiment of FIG. 39, the motor may have an infinitely variable speed adjustment (or more than just a two speed adjustment) capability that the integrated data of smoke density and time duration of that smoke density may be used with. In this manner, the controller 240 may execute an algorithm, or a look-up table of predetermined settings of density and time duration, to make the variable speed adjustments of the pump motor 207. Thus, during a surgical procedure the smoke detection sensor may detect smoke in the gas in the peritoneum and the controller 240 may correlate the amount of detected smoke to control a rate of circulation to filter out the smoke within the pneumoperitoneum in order to provide an ideal surgical field at all times. The predetermined, or user selectable ranges, of smoke density (e.g. light attenuation) and pump motor speed or flow rate may be stored in volatile or non-volatile memory within the controller 240 or accessible to the controller 240.

The embodiments of adjusting pump speed to circulate or more quickly circulate gas when smoke has been detected above a particular threshold may not always provide fast enough smoke removal capabilities. Referring again to FIG. 26, the system 2600 may optionally include a valve 2604 positioned on the gas flow path output 225 to switch from a closed, recirculating system that filters out smoke, to an exhaust mode where smoke-filled gas can quickly be shunted to a suction exhaust tube 2606 for higher smoke removal rates.

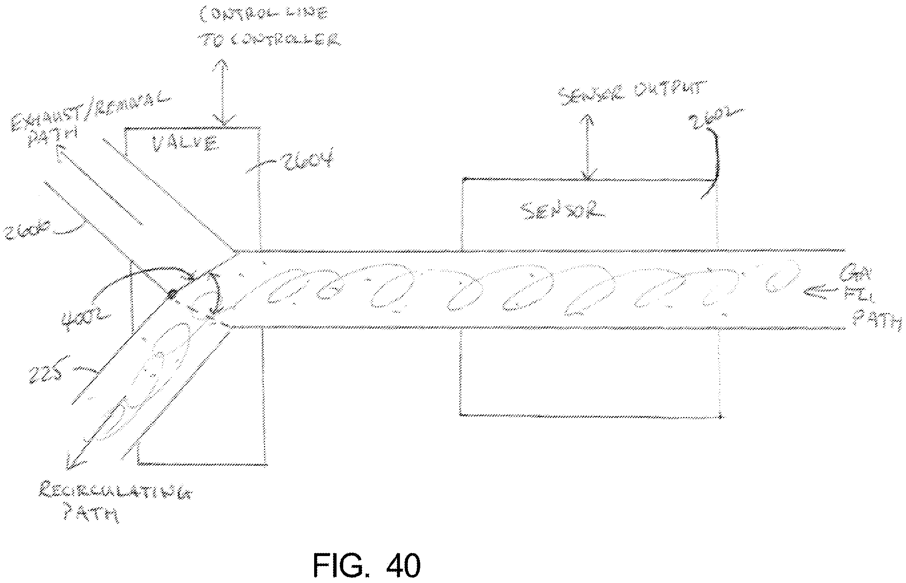

As shown in FIG. 40, the valve 2604 may be controllable by the controller 240 to swing a valve door 4002 from a position that routes all the gas through the tubing of the closed system recirculating path to a second position (dotted lines) where the gas is instead diverted to the suction exhaust tube 2606. The suction exhaust tube defines an exhaust/removal path out to the suction source 2608, as described previously. Alternatively, the valve 2604 may receive an activation signal directly from the smoke detection sensor 2602 in alternative embodiments, where a high enough output signal directly from the sensor 2602 causes the valve to open the suction exhaust path. The example of FIG. 40 focuses on the embodiment of FIG. 26 with the smoke sensor located in a tube, however the embodiment of FIG. 29, with the smoke detection sensor 2902 in the pump or pump cartridge, may also be modified to include a suction exhaust tube, valve and suction source in either the gas input or output tube, similar to that shown in FIG. 26, and operate as described in FIG. 40.

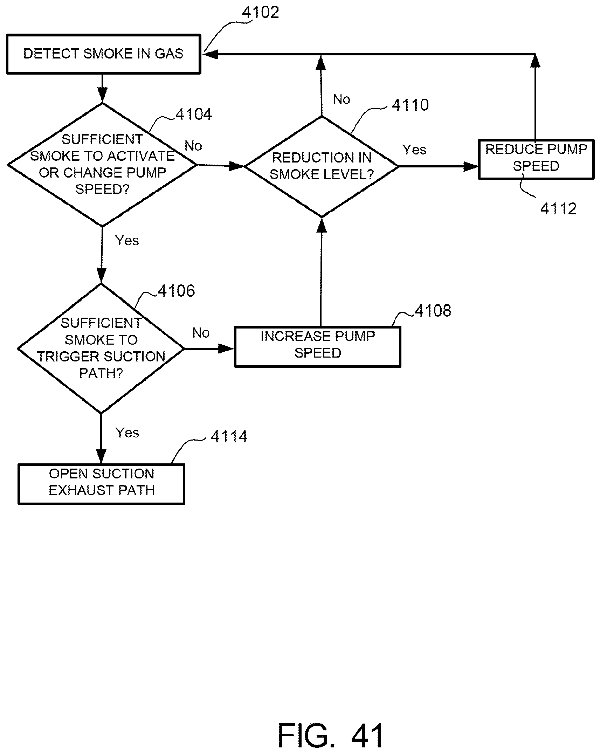

As illustrated in FIG. 41, in some implementations of the gas recirculation system 2600 with gas detection, the suction exhaust tube 2606 and valve 2604 may be used at different levels of smoke detection to provide a faster smoke removal option than may be available with increase recirculation pump speed through smoke removal filters. FIG. 41 illustrates removing smoke in a system 2600 that includes both adjustable gas recirculation speed in a closed gas recycling mode and a selectable suction exhaust to expel smoke filled gas outside of the closed recirculation system. The first step in the process is detecting smoke in the gas using the gas sensor 2602 (at 4102). It is then determining whether there is sufficient smoke present to activate (in an on/off embodiment as discussed previously) or increase the speed of (in embodiments where the pump is always on, whether having limited speed steps or infinitely variable) the motor of the pump (at 4104). If the smoke amount is sufficiently high to activate or increase the pump speed, but does not exceed the higher threshold that would require use of the suction exhaust path (at 4106), then the controller 240 may activate or increase the pump motor speed as appropriate (at 4108). If the amount of smoke detected is sufficient to trigger the higher threshold suction path (at 4106), then the controller 240 will instead activate the valve 2604 to vent the gas and smoke through a suction source 2608 and bypass the closed recirculation path of the system 2600 (at 4114). The controller may use subsequent smoke sensor 2602 measurements (at 4104, 4110) to then reduce or turn off the pump motor 207, when the amount of smoke is reduced (at 4112).

As noted in the prior smoke sensing embodiments, different smoke measurement criteria may be used. In one implementation, the amount of smoke may be a single instantaneous measurement. Alternatively, the smoke measurement may be a combination of the amount of smoke measured over a duration of time, where an average amount over a given time is used. Additionally, a combination of different smoke densities and associated durations of those smoke densities may trigger increased or reduced pump motor speed, or may meet the threshold for triggering the use by the controller of the suction exhaust path.

Referring to the light attenuation and pump motor speed (or flow rate) example above, the threshold for opening the valve 2604 to vent gas through a suction source may be an amount of light attenuation greater than that set for the highest motor speed setting (e.g. 20% light attenuation for the valve to exhaust to the suction source compared to the 10% attenuation to activate the highest motor speed in the prior example). Alternatively or in combination, the threshold for activating the valve to exhaust the gas through the suction source may include threshold duration of a percentage light attenuation sensed. For example, if the duration of a predetermined sensed light attenuation due to smoke in the gas exceeds a specific time limit the controller 240 may trigger the valve 2604 to connect the suction source 2608 and bypass the pump to exhaust the smoke-filled gas via the suction source.

It is further contemplated that other sensed criteria regarding the gas from the peritoneum may be used in combination with, or separately from, the gas density and/or duration measurements. For example, the system 2600, 2900 may include a temperature sensor, placed in the pneumoperitoneum or along the path of the suction/filtration tubing which will allow for real-time determination of the temperature of the pneumoperitoneum. This temperature measurement can then cause the controller to activate the movement of the gas as required, to cool down the gas. The temperature sensor may be integrated into the pump 205, added to the smoke detection sensor 2602, 2902 mounted on another instrument being used in the laparoscopic procedure, or may be a standalone sensor placed in the tubing of the recirculation system. The temperature sensor may be used to detect a thermal transfer rate difference in the gas that relates to the amount of smoke present. For example, the thermal transfer rate of a smoke-filled carbon dioxide gas may have different heat flux properties as compared to smoke-free carbon dioxide. As a more specific example, carbon dioxide has a K factor of 0.658 relative to nitrogen, while carbon monoxide has a K factor of 1.00 relative to nitrogen (where K= 1/((gas density) x (coefficient of specific heat)). Thus, keeping the flow rate constant but increasing the concentration of carbon monoxide would reduce the thermal flux.

Other sensors discussed herein, and other sensor technologies that are contemplated for use alone or in combination, include chemical sensing for detection of expected chemical composition of surgical smoke (e.g. carbon monoxide), sonic or ultrasonic sensors, magnetic sensors, chromatographic sensors, and cautery device use signals and activity sensors (e.g. a sensor tuned to a unique audio signature that a cautery device is known to emit when in operation may be detected at a microphone-type sensor and used to trigger increased pump speed in the gas recirculation system). For example, with respect to an audio sensor, an electrosurgical unit which sounds (generates) a continuous 3kHz audio tone when it is actively cauterizing may be detected by an audio receiver circuit which utilizes bandpass filtering tuned to the same frequency. The duration of the tone may be measured to determine the duration of cauterization. Alternately, an electrosurgical unit which sounds a three-pulse tone of 3kHz when cauterization begins and then another when it is stopped may be similarly detected and the time between the two three-pulse tone sequences may be calculated to determine the duration of cauterization. The resulting determination of cauterization duration may be used to modulate the motor speed, or to both modulate motor speed and trigger a smoke evacuation bypass at respective different thresholds, analogous to how the smoke density may be used in the smoke sensor embodiments described above.

Different locations of the smoke detection sensor or sensors in the smoke removal system, for example in or along the tubing, in or along the pump or pump cartridge, or in the connectors, all have different potential advantages over each other. In the case of placement in or along the tubing relative to placement in one of the luer connectors advantages may include reduced potential for damage of the sensor while the user is manually manipulating (disconnecting/connecting) the luer, and reducing the amount of equipment at the surgical site for improved usability and ergonomics. Also, placement in or along the tubing relative to placement in the pump may include similar advantages as those for placement in the luer, such as reduced response time and/or increased sensitivity due to closer proximity to the cauterization location, and may potentially reduce possible sensor wear by physically isolating the sensor from the pump's mechanical oscillations.

While various embodiments of the invention have been described, it will be apparent to those of ordinary skill in the art that many more embodiments and implementations are possible within the scope of the invention. The elements of the various embodiments disclosed may be combined and adapted to create a system with some or all of the operating characteristics and advantages of the embodiments. Any such combinations are herein disclosed in this application.

## Claims

1. A gas recirculation system (2600) for use in managing a flow of gas in an endoscopic surgical procedure, the system comprising:
a first tube (220) in fluid communication with a gas input connection, wherein the first tube (220) is configured to be connectable to surgical equipment that is insertable into a peritoneal cavity; and
a second tube (225) in fluid communication with a gas output connection,
wherein the second tube (225) is configured to be connectable to surgical equipment that is insertable into a peritoneal cavity;
a pump (205) having a motor (207), wherein the pump (205) is configured to draw gas into the gas input connection from a peritoneal cavity through the first tube (220) and to discharge gas out of the gas output connection and into a peritoneal cavity through the second tube (225);
a smoke detection sensor (2602, 2702, 2802, 3200) positioned at a location along a gas flow path defined by the first tube (220), the pump (205) and the second tube (225) and configured to measure an amount of smoke present in the gas;
a controller (240) configured to receive an output signal from the smoke detection sensor (2602, 2702, 2802, 3200) representative of an amount of smoke detected; and adjust a speed of the motor (207) of the pump (205) in response to the amount of smoke detected, and
a valve (2604) positioned along the first tube (220) between the smoke detection sensor (2602, 2702, 2802, 3200) and the pump (205), the valve (2604) adjustable via the controller (240) to bypass the pump (207) and direct the flow of gas from the first tube (220) to a suction exhaust tube (2606);
wherein the controller (240) is configured to adjust the valve (2606) to direct the flow of gas to the exhaust suction tube (2606) in response to the output signal indicating an amount of smoke above an evacuation threshold.

2. The gas recirculation system (2600) of claim 1, wherein the smoke detection sensor (2602, 2702, 2802, 3200) is positioned along the first tube (220).

3. The gas recirculation system (2600) of claim 1, wherein the smoke detection sensor (2602, 2702, 2802, 3200) is positioned along the second tube (225).

4. The gas recirculation system (2600) of claim 1, wherein the smoke detection sensor (2902) is positioned in the pump (205).

5. The gas recirculation system (2600) of claim 1, wherein the smoke detection sensor (2602, 2702, 2802, 3200) comprises an ionizing smoke detector circuit.

6. The gas recirculation system (2600) of claim 1, wherein the smoke detection sensor (2602, 2702, 2802, 3200) comprises an optical sensor.

7. The gas recirculation system (2600) of claim 6, wherein the optical sensor comprises a light source (2706, 2806) and a photoreceptor (2708, 2808, 2809) positioned to receive light emitted from the light source.

8. The gas recirculation system (2600) of claim 7, wherein the photoreceptor (2708) is positioned at an angle with respect to the light source (2706) such that the photoreceptor (2708) can only receive light from the light source (2706) that has been reflected from smoke in the gas.

9. The gas recirculation system (2600) of claim 1, wherein the controller (240) is configured to increase the speed of the motor (207) of the pump (205) in response to an increase in the amount of smoke detected.

10. The gas recirculation system (2600) of claim 1, wherein the smoke detection sensor (2702) is configured to wirelessly transmit the output signal to the controller (240) via wireless connection (2710).

11. The gas recirculation system (2600) of claim 7, wherein the light source (2706) is an infrared light source.

12. The gas recirculation system (2600) of claim 1, further comprising:
a filter (230) positioned along the second tube (225), the filter (230) configured to remove smoke from the flow of gas through the second tube (225).

13. A gas recirculation system (2600) according to anyone of claims 1 to 12, wherein the controller (240) is further configured to, in response to determining that an amount of smoke detected exceeds an evacuation threshold, direct gas from the first tube (220) to a suction source (2608).

14. The gas recirculation system (2600) according to anyone of claims 1 to 13, wherein the pump (205) comprises:
a pump cartridge (206) coupled to the motor (207), wherein
the pump cartridge (206) is detachable from the motor (207), connects with the first tube (220) at the gas input connection, and with the second tube (225) at the gas output connection; and
the pump cartridge (206) is sealed such that a gas within the pump cartridge (206) cannot contact the motor (207).

## Patentansprüche

1. Gasumlauf-System (2600) zum Einsatz beim Leiten eines Stroms von Gas bei einem chirurgischen Endoskopie-Eingriff, wobei das System umfasst:
eine erste Röhre (220), die in Fluidverbindung mit einer Gas-Eingangsverbindung steht, wobei die erste Röhre (220) so ausgeführt ist, dass sie mit einem chirurgischen Instrument verbunden werden kann, das in eine Bauchhöhle eingeführt werden kann; sowie
eine zweite Röhre (225), die in Fluidverbindung mit einer Gas-Ausgangsverbindung steht,
wobei die zweite Röhre (225) so ausgeführt ist, dass sie mit einem chirurgischen Instrument verbunden werden kann, das in eine Bauchhöhle eingeführt werden kann;
eine Pumpe (205) mit einem Motor (207), wobei die Pumpe (205) so ausgeführt ist, dass sie Gas aus einer Bauchhöhle über die erste Röhre (220) in die Gas-Eingangsverbindung hinein ansaugen kann und Gas aus der Gas-Ausgangsverbindung Gas über die zweite Röhre (225) in eine Bauchhöhle hinein ausstoßen kann;
einen Raucherfassungs-Sensor (2602, 2702, 2802, 3200), der an einer Position entlang eines Gas-Strömungsweges angeordnet ist, der durch die erste Röhre (220), die Pumpe (205) sowie die zweite Röhre (225) gebildet wird, und so ausgeführt ist, dass er eine in dem Gas vorhandene Menge an Rauch misst;
eine Steuerungseinrichtung (240), die so ausgeführt ist, dass sie ein Ausgangssignal von dem Raucherfassungs-Sensor (2602, 2702, 2802, 3200) empfängt, das repräsentativ für eine erfasste Menge an Rauch ist, und eine Geschwindigkeit des Motors (207) der Pumpe (205) in Reaktion auf die erfasste Menge an Rauch einstellt, sowie
ein Ventil (2604), das entlang der ersten Röhre (220) zwischen dem Raucherfassungs-Sensor (2602, 2702, 2802, 3200) und der Pumpe (205) angeordnet ist, wobei das Ventil (2604) über die Steuerungseinrichtung (240) so eingestellt werden kann, dass die Pumpe (207) umgangen wird und der Strom von Gas von der ersten Röhre (220) zu einer Ansaug-Ableitröhre (2606) geleitet wird;
wobei die Steuerungseinrichtung (240) so ausgeführt ist, dass sie das Ventil (2606) so einstellt, dass der Strom von Gas in Reaktion darauf, dass das Ausgangssignal eine Menge an Rauch über einem Evakuierungs-Schwellenwert anzeigt, zu der Ansaug-Ableitröhre (2606) geleitet wird.

2. Gasumlauf-System (2600) nach Anspruch 1, wobei der Raucherfassungs-Sensor (2602, 2702, 2802, 3200) entlang der ersten Röhre (220) angeordnet ist.

3. Gasumlauf-System (2600) nach Anspruch 1, wobei der Raucherfassungs-Sensor (2602, 2702, 2802, 3200) entlang der zweiten Röhre (225) angeordnet ist.

4. Gasumlauf-System (2600) nach Anspruch 1, wobei der Raucherfassungs-Sensor (2902) in der Pumpe (205) angeordnet ist.

5. Gasumlauf-System (2600) nach Anspruch 1, wobei der Raucherfassungs-Sensor (2602, 2702, 2802, 3200) einen ionisierenden Raucherfassungs-Kreis umfasst.

6. Gasumlauf-System (2600) nach Anspruch 1, wobei der Raucherfassungs-Sensor (2602, 2702, 2802, 3200) einen optischen Sensor umfasst.

7. Gasumlauf-System (2600) nach Anspruch 6, wobei der optische Sensor eine Lichtquelle (2706, 2806) sowie einen Fotorezeptor (2708, 2808, 2809) umfasst, der so angeordnet ist, dass er von der Lichtquelle emittiertes Licht empfängt.

8. Gasumlauf-System (2600) nach Anspruch 7, wobei der Fotorezeptor (2708) in einem Winkel in Bezug auf die Lichtquelle (2706) angeordnet ist, in dem der Fotorezeptor (2708) nur Licht von der Lichtquelle (2706) empfangen kann, das von Rauch in dem Gas reflektiert worden ist.

9. Gasumlauf-System (2600) nach Anspruch 1, wobei die Steuerungseinrichtung (240) so ausgeführt ist, dass sie die Geschwindigkeit des Motors (207) der Pumpe (205) in Reaktion auf eine Zunahme der erfassten Menge an Rauch erhöht.

10. Gasumlauf-System (2600) nach Anspruch 1, wobei der Raucherfassungs-Sensor (2702) so ausgeführt ist, dass er das Ausgangssignal über eine Drahtlosverbindung (2710) drahtlos zu der Steuerungseinrichtung (240) sendet.

11. Gasumlauf-System (2600) nach Anspruch 7, wobei die Lichtquelle (2706) eine Infrarot-Lichtquelle ist.

12. Gasumlauf-System (2600) nach Anspruch 1, das des Weiteren umfasst:
einen Filter (230), der entlang der zweiten Röhre (225) angeordnet ist, wobei der Filter (230) so ausgeführt ist, dass er Rauch aus dem Strom von Gas über die zweite Röhre (225) entfernt.

13. Gasumlauf-System (2600) nach einem der Ansprüche 1 bis 12, wobei die Steuerungseinrichtung (240) des Weiteren so ausgeführt ist, dass sie in Reaktion darauf, dass festgestellt wird, dass eine erfasste Menge an Rauch einen Evakuierungs-Schwellenwert übersteigt, Gas von der ersten Röhre (22) zu einer Ansaugquelle (2608) leitet.

14. Gasumlauf-System (2600) nach einem der Ansprüche 1 bis 13, wobei die Pumpe (205) umfasst:
einen Pumpeneinsatz (206), der mit dem Motor (207) gekoppelt ist, wobei
der Pumpeneinsatz (206) von dem Motor (207) getrennt werden kann, mit der ersten Röhre (220) an der Gas-Eingangsverbindung und mit der zweiten Röhre (225) an der Gas-Ausgangsverbindung verbunden ist; und
der Pumpeneinsatz (206) so abgedichtet ist, dass ein Gas im Inneren des Pumpeneinsatzes (206) nicht mit dem Motor (207) in Kontakt kommen kann.

## Revendications

1. Système de recirculation de gaz (2600) destiné à être utilisé pour gérer un flux de gaz dans une intervention chirurgicale endoscopique, le système comprenant :
un premier tube (220) en communication fluidique avec une connexion d'entrée de gaz, dans lequel le premier tube (220) est configuré pour pouvoir être connecté à un équipement chirurgical insérable dans une cavité péritonéale ; et
un deuxième tube (225) en communication fluidique avec une connexion de sortie de gaz, dans lequel le deuxième tube (225) est configuré pour pouvoir être connecté à un équipement chirurgical insérable dans une cavité péritonéale ;
une pompe (205) comportant un moteur (207), dans lequel la pompe (205) est configurée pour aspirer du gaz dans la connexion d'entrée de gaz depuis une cavité péritonéale via le premier tube (220) et pour évacuer le gaz hors de la connexion de sortie de gaz et dans une cavité péritonéale via le deuxième tube (225) ;
un capteur de détection de fumée (2602, 2702, 2802, 3200) positionné à un emplacement le long d'une voie d'écoulement de gaz définie par le premier tube (220), la pompe (205) et le deuxième tube (225), et configuré pour mesurer une quantité de fumée présente dans le gaz ;
un contrôleur (240) configuré pour recevoir un signal de sortie du capteur de détection de fumée (2602, 2702, 2802, 3200) représentatif d'une quantité de fumée détectée, et pour ajuster la vitesse du moteur (207) de la pompe (205) en réponse à la quantité de fumée détectée, et
une vanne (2604) positionnée le long du premier tube (220) entre le capteur de détection de fumée (2602, 2702, 2802, 3200) et la pompe (205), la vanne (2604) étant ajustable via le contrôleur (240) pour contourner la pompe (207) et diriger le flux de gaz depuis le premier tube (220) vers un tube d'aspiration d'échappement (2606) ;
dans lequel le contrôleur (240) est configuré pour ajuster la vanne (2604) afin de diriger le flux de gaz vers le tube d'aspiration d'échappement (2606) en réponse à une indication par le signal de sortie d'une quantité de fumée supérieure à un seuil d'évacuation.

2. Système de recirculation de gaz (2600) selon la revendication 1, dans lequel le capteur de détection de fumée (2602, 2702, 2802, 3200) est positionné le long du premier tube (220).

3. Système de recirculation de gaz (2600) selon la revendication 1, dans lequel le capteur de détection de fumée (2602, 2702, 2802, 3200) est positionné le long du deuxième tube (225).

4. Système de recirculation de gaz (2600) selon la revendication 1, dans lequel le capteur de détection de fumée (2902) est positionné dans la pompe (205).

5. Système de recirculation de gaz (2600) selon la revendication 1, dans lequel le capteur de détection de fumée (2602, 2702, 2802, 3200) comprend un circuit de détection de fumée ionisant.

6. Système de recirculation de gaz (2600) selon la revendication 1, dans lequel le capteur de détection de fumée (2602, 2702, 2802, 3200) comprend un capteur optique.

7. Système de recirculation de gaz (2600) selon la revendication 6, dans lequel le capteur optique comprend une source de lumière (2706, 2806) et un photorécepteur (2708, 2808, 2809) positionné pour recevoir la lumière émise par la source de lumière.

8. Système de recirculation de gaz (2600) selon la revendication 7, dans lequel le photorécepteur (2708) est positionné à un angle par rapport à la source de lumière (2706) de telle sorte que le photorécepteur (2708) ne peut recevoir que la lumière provenant de la source de lumière (2706) qui a été réfléchie par la fumée présente dans le gaz.

9. Système de recirculation de gaz (2600) selon la revendication 1, dans lequel le contrôleur (240) est configuré pour augmenter la vitesse du moteur (207) de la pompe (205) en réponse à une augmentation de la quantité de fumée détectée.

10. Système de recirculation de gaz (2600) selon la revendication 1, dans lequel le capteur de détection de fumée (2702) est configuré pour transmettre sans fil le signal de sortie au contrôleur (240) via une connexion sans fil (2710).

11. Système de recirculation de gaz (2600) selon la revendication 7, dans lequel la source de lumière (2706) est une source de lumière infrarouge.

12. Système de recirculation de gaz (2600) selon la revendication 1, comprenant en outre :
un filtre (230) positionné le long du deuxième tube (225), le filtre (230) étant configuré pour éliminer la fumée du flux de gaz via le deuxième tube (225).

13. Système de recirculation de gaz (2600) selon l'une quelconque des revendications 1 à 12, dans lequel le contrôleur (240) est en outre configuré pour, en réponse à la détermination du fait qu'une quantité de fumée détectée dépasse un seuil d'évacuation, diriger le gaz depuis le premier tube (220) vers une source d'aspiration (2608).

14. Système de recirculation de gaz (2600) selon l'une quelconque des revendications 1 à 13, dans lequel la pompe (205) comprend :
une cartouche de pompe (206) couplée au moteur (207), dans lequel
la cartouche de pompe (206) est détachable du moteur (207), et se connecte au premier tube (220) via la connexion d'entrée de gaz et au deuxième tube (225) via la connexion de sortie de gaz ; et
la cartouche de pompe (206) est scellée de telle sorte qu'un gaz présent à l'intérieur de la cartouche de pompe (206) ne puisse pas entrer en contact avec le moteur (207).
